# EUROPEAN PATENT SPECIFICATION

(11) **EP 4 655 305 B1**
(45) Date of publication and mention of the grant of the patent: **13.05.2026**
(21) Application number: 24722467.8
(22) Date of filing: 28.03.2024
(51) Int. Cl.: C07H 23/00, C07H 17/02, A61P 31/18, A61P 35/00

(54) **RUTHENIUM COMPLEXES FOR INHIBITION OF GALECTINS, METHOD OF THEIR PREPARATION, AND USE THEREOF**
RUTHENIUMKOMPLEXE ZUR HEMMUNG VON GALECTINEN, VERFAHREN ZU IHRER HERSTELLUNG UND IHRE VERWENDUNG
COMPLEXES DE RUTHÉNIUM POUR L'INHIBITION DE GALECTINES, LEUR PROCÉDÉ DE PRÉPARATION ET LEUR UTILISATION

(30) Priority: 04.04.2023 CZ 20230132
(43) Date of publication of application: 03.12.2025
(73) Proprietor: Ustav Chemickych Procesu AV CR, V.V.I., 165 02 Praha 6 - Suchdol (CZ); Masarykuv Onkologicky Ustav, 656 53 Brno (CZ)
(72) Inventor: HRSTKA, Roman, 66467 Bratcice (CZ); HOLCAKOVA, Jitka, 66501 Rosice (CZ); KARBAN, Jindrich, 25263 Roztoky (CZ); HAMALA, Vojtech, 16000 Praha 6 (CZ); KURFIRT, Martin, 46826 Hut Pencin (CZ)
(74) Representative: Harber IP s.r.o.
(86) International application number: PCT/CZ2024/050020
(87) International publication number: WO 2024/208383

(56) References cited:
- WO-A1-2014/078655
- WO-A1-2018/011093
- TSUSHIMA MICHIHIKO ET AL: "Catalyst-proximity protein chemical labelling on affinity beads targeting endogenous lectins", CHEMICAL COMMUNICATIONS, vol. 55, no. 88, 31 October 2019 (2019-10-31), UK, pages 13275 - 13278, XP093132049, ISSN: 1359-7345, DOI: 10.1039/C9CC05231C

## Description

### Field of Art

The present invention relates to ruthenium complexes that exhibit significant affinity and selectivity towards human galectin 1 and thus have the potential to become active pharmaceutical ingredients inhibiting galectin 1 for use, for example, in the treatment of cancerous diseases, fibrotic diseases, and HIV infection.

### Background Art

Galectins are proteins from the lectin group defined by sequence homology and the ability to non-covalently bind β-galactosides. 16 types of galectins are described in mammals, with 13 occurring in humans. All types of galectins have an evolutionarily conserved carbohydrate recognition domain (CRD) composed of approximately 130 amino acids arranged in a β-sandwich structure formed by five- and six-membered antiparallel β-sheet structures.

Based on the type of quaternary structure, galectins are divided into 3 groups:
1. Prototypical galectins that contain one CRD which spontaneously forms a non-covalent homodimer under physiological conditions. Galectins 1, 2, 5, 7, 10, 11, 13, 14, 15, and 16 belong to this group.
2. Tandem galectins that possess two CRDs linked by a short peptide linker. Galectins 4, 6, 8, 9, and 12 belong to this group.
3. Chimeric galectin 3 that consists of CRD and an N-terminal polypeptide chain of approximately 120 amino acids, enabling the formation of non-covalently bound oligomeric structures.

Typical endogenous galectin ligands are disaccharides N-acetyllactosamine (Galβ1-4GlcNAc) and lactose (Galβ1-4Glc). The binding between galectin and disaccharides occurs in a relatively shallow binding site on highly conserved binding subsites C and D. The interaction is mediated by 6 amino acids in the binding cavity by hydrogen bonds between these amino acids (typically histidine, arginine, and asparagine) and the disaccharide hydroxyl groups, as well as by CH-π interactions between the nonpolar α-face of β-galactoside and the tryptophan amino acid. Segments A, B, and E, neighboring segments C and D, exhibit lower conservation levels and display greater divergence, particularly influenced by the specific type of galectin. These segments participate in interactions with ligands larger than disaccharides.

Galectins in the body perform various biological functions, including maintaining cellular homeostasis, regulating cellular apoptosis, or angiogenesis. Additionally, changes in galectin expression (often involving overproduction) are associated with many pathological conditions: cancer, HIV infection, pulmonary fibrosis, inflammation, or autoimmune diseases. In tumor cells and tissues, galectins are partly responsible for cellular malignant transformation and subsequent evasion of immune surveillance, leading to tumor progression, including enhanced invasiveness, angiogenesis, migration of tumor cells, and metastasis formation. Therefore, galectins are currently considered a promising therapeutic target.

### Natural and Synthetic Galectin Ligands

In addition to the mentioned disaccharides, galectins bind to endogenous ligands comprising structures further modified (e.g., Lewis antigens) and oligomerized (e.g., poly-N-acetyllactosamines). Synthetic saccharide ligands with binding to galectins include, for example, α-thiogalactosides and thiodigalactosides (Cummings R. D.; Liu F. T.; Rabinovich G. A.; et al. Galectins. In: Varki A.; Cummings R. D.; Esko J. D.; et al., editors. Essentials of Glycobiology [Internet]. 4th edition. Cold Spring Harbor (NY): Cold Spring Harbor Laboratory Press; 2022. Chapter 36. Available from: https://www.ncbi.nlm.nih.gov/books/NBK579987/ doi: 10.1101/glycobiology.4e.36). Hydroxyl groups that do not engage in interactions with galectin can undergo modification to enhance either the affinity or selectivity of the interaction. (Denavit, V.; Lainé, D.; Tremblay, T.; St-Gelais, J.; Giguère, D., Synthetic inhibitors of galectins: Structures and syntheses. Trends in Glycoscience and Glycotechnology 2018, 30, (172), SE21-SE40; Kurfiřt, M.; Dračínský, M.; Červenková Št'astná, L.; Cuřínová, P.; Hamala, V.; Hovorková, M.; Bojarová, P.; Karban, J., Selectively Deoxyfluorinated N-Acetyllactosamine Analogues as 19F NMR Probes to Study Carbohydrate-Galectin Interactions. Chemistry-A European Journal 2021, 27 (51), 13040-13051). Substituting certain non-binding hydroxyls with aromatic structures bearing halogen substituents leads to the creation of compounds with markedly higher affinity for galectins compared to their natural ligands. This enhanced affinity is attributed to both π-π interactions between aromatic structures and aromatic amino acids within galectins, as well as interactions between halogens and amino acid polar groups. Synthetic inhibitors with pharmacological promise generally exhibit significantly greater affinity for galectin-3 than for galectin-1. Conversely, inhibitors with high affinity and selectivity for galectin-1 have not yet been developed. Among the strongest and clinically most promising inhibitors of galectins are galectin-3 inhibitors: GB139 and GB1211, which has been up to the phase 2 clinical trials for the treatment of pulmonary fibrosis, liver damage, non-small cell lung carcinoma, myelofibrosis, and head and neck squamous cell carcinoma (Hirani, N.; MacKinnon, A. C.; Nicol, L.; Ford, P.; Schambye, H.; Pedersen, A.; Nilsson, U. J.; Leffler, H.; Sethi, T.; Tantawi, S., Target inhibition of galectin-3 by inhaled TD139 in patients with idiopathic pulmonary fibrosis. European Respiratory Journal 2021, 57 (5), 2002559; Zetterberg, F. R.; MacKinnon, A.; Brimert, T.; Gravelle, L.; Johnsson, R. E.; Kahl-Knutson, B.; Leffler, H.; Nilsson, U. J.; Pedersen, A.; Peterson, K., Discovery and Optimization of the First Highly Effective and Orally Available Galectin-3 Inhibitors for Treatment of Fibrotic Disease. Journal of Medicinal Chemistry 2022, 65 (19), 12626-12638).

The chemical structure of compound GB139 and its analogs is derived from a thiodigalactoside framework with modifications on both galacto units at carbon C3. The substitution of C3 hydroxyls with aromatic substituents notably enhances affinity to galectins, particularly galectin-3. Aromatic substituents may be linked to the thiodigalactoside framework through ether, ester, or amide bonds, or via a 1,2,3-triazole linkage formed through azide-alkyne cycloaddition reaction, as demonstrated in the case of GB139. The structures of thiodigalactoside and GB139 are shown in Scheme 1 below.

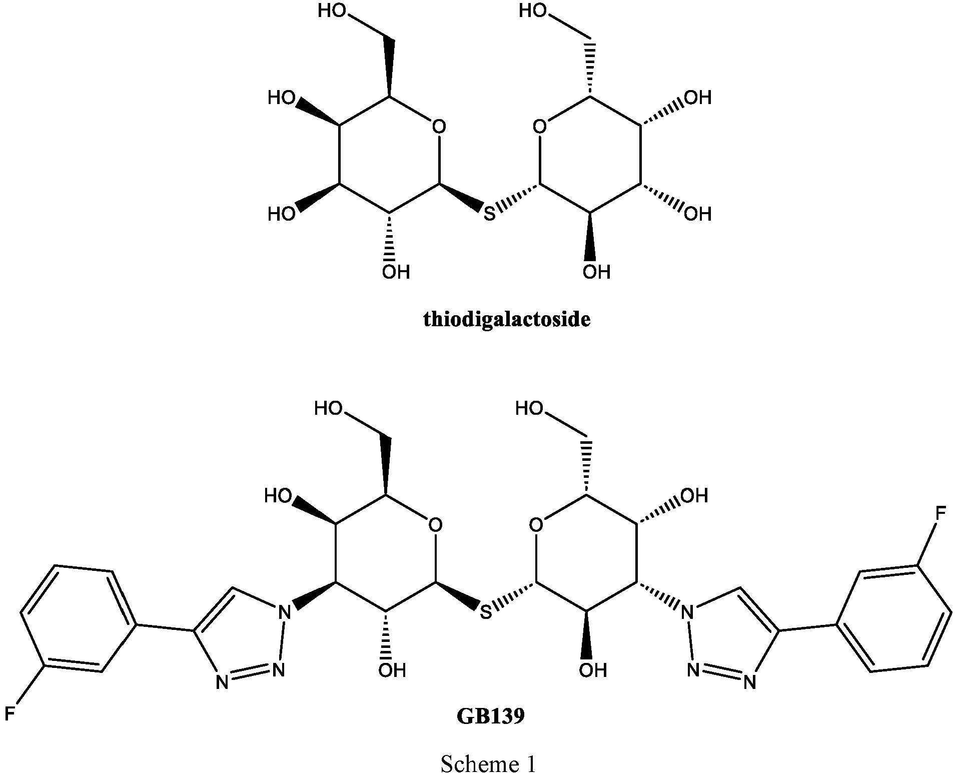

### Human Galectin 1

Galectin 1, encoded by the LGASL1 gene, is distributed across the cytoplasm, cell nucleus, and extracellular space. As a prototypical galectin, it predominantly exists in the extracellular space as a homodimer, facilitating its involvement in cellular signaling, adhesion, and various cellular processes. Galectin 1 plays a pivotal role in immune system modulation, regulating B cell proliferation and maintaining T cell homeostasis, with elevated concentrations capable of inducing apoptosis in T cells. Furthermore, the overproduction of galectin-1 influences phagocytosis and the activation of peritoneal macrophages.

Elevated levels of galectin 1 have been detected in various cancers including prostate, lung, breast, colon, urinary tract, kidney, lymph nodes, skin, pancreas, upper gastrointestinal tract, thyroid gland, stomach, brain, spinal cord, ovaries, liver, white blood cells, and uterus cancers. The overexpression of galectin-1 correlates with uncontrolled tumor growth and the formation of metastases, promotes angiogenesis within tumor tissues, and enhances tumor resistance to immune surveillance. In both *in vitro* and *in vivo* experiments, suppression of galectin 1 expression in tumor cells, achieved through various methods including the use of synthetic inhibitors, has been shown to diminish the metastatic potential of tumors and enhance the effectiveness of other anti-cancer treatments such as radiotherapy or chemotherapy. (Astorgues-Xerri, L.; Riveiro, M. E.; Tijeras-Raballand, A.; Serova, M.; Neuzillet, C.; Albert, S.; Raymond, E.; Faivre, S., Unraveling galectin-1 as a novel therapeutic target for cancer. Cancer Treatment Reviews 2014, 40 (2), 307-319). For example, inhibition of galectin 1 increased the sensitivity of ovarian carcinoma cells to the anti-cancer drug Paclitaxel (Shih, T.-C.; Liu, R.; Fung, G.; Bhardwaj, G.; Ghosh, P. M.; Lam, K. S., A Novel Galectin-1 Inhibitor Discovered through One-Bead Two-Compound Library Potentiates the Antitumor Effects of Paclitaxel in vivo. Molecular Cancer Therapeutics 2017, 16 (7), 1212-1223). Suppression of galectin 1 expression also increased the sensitivity of breast cancer cells to the anti-cancer drugs Paclitaxel and Adriamycin (Wang, F.; Lv, P.; Gu, Y.; Li, L.; Ge, X.; Guo, G., Galectin-1 knockdown improves drug sensitivity of breast cancer by reducing P-glycoprotein expression through inhibiting the Raf-1/AP-1 signaling pathway. Oncotarget 2017, 8 (15), 25097). Galectin 1 inhibition increases the sensitivity of tumor cells to radiotherapy while reducing radiation-induced lymphopenia (Welsh, J. W.; Seyedin, S. N.; Cortez, M. A.; Maity, A.; Hahn, S. M., Galectin-1 and Immune Suppression during Radiotherapy. Clinical Cancer Research 2014, 20 (24), 6230-6232).

The presence of galectin 1 also facilitates the adhesion of human immunodeficiency virus type 1 (HIV-1) to macrophages, significantly increasing the risk of infection and accelerating its spread within the infected organism. Galectin 1 homodimers can bind to carbohydrate structures on both the surface of macrophages, using one of its two CRDs, and on the surface of HIV-1, using the other CRD. This facilitates the interaction between the CD4 glycoprotein of the infected cell and the HIV glycoprotein gb120, essential for virus fusion with the infected cell. Notably, this effect has only been demonstrated for galectin 1, not for galectin 3. Moreover, HIV-1 infection in T-lymphocytes triggers aberrant glycosylation of membrane proteins, resulting in the accumulation of (poly)lactosamine structures, which are natural ligands of galectin 1. Consequently, the interaction of (poly)lactosamine structures on infected cells with galectin 1 induces apoptosis of both infected and uninfected T lymphocytes, leading to the release of virions into the surrounding environment.

WO 2018/011093 A1 discloses Galectin-1 inhibitors based on α-D-galactosides that have shown high affinity and selectivity for galectin-1, and are potential candidates for treatment of disorders such as cancers, fibrosis, scarring, keloid formation; aberrant scar formation; surgical adhesions; pathological angiogenesis; eye diseases; HIV-1 diseases; inflammation or transplant rejection in mammals.

WO 2014/078655 A1 discloses Galectin-1 inhibitors comprising a galactopyranose ring, substituted with aromatic rings connected to said galactopyranose ring via triazole linker, for use in inhibiting ocular angiogenesis or ocular fibrosis.

Tsushima Michihiko et al., "Catalyst-proximity protein chemical labelling on affinity beads targeting endogenous lectins", Chem. Comm., 2019, vol. 55, no. 88, p. 13275-13278, discloses lactose conjugated Ru(bpy)₃²⁺ complex as photocatalyst (protein chemical labelling).

Hence, the ongoing requirement for novel and potent galectin inhibitors, specifically those exhibiting selectivity towards galectin 1, remains.

### Disclosure of the Invention

The present invention relates to compounds (ruthenium complexes) discovered to selectively inhibit galectin 1. These compounds thus hold significance for the pharmaceutical industry, facilitating the development of new drugs, as well as for galectin function studies in both basic and applied research, encompassing *in vitro* and *in vivo* experimentation. Diverging from previously known galectin inhibitors, the compounds according to the present invention exhibit selectivity towards galectin 1, enabling its inhibition in the presence of other galectins. Unlike the planar aromatic rings present in prior art inhibitors like GB139 and its analogs, the organometallic complexes according to the present invention possess a three-dimensional (3D) structure. This structural complexity, challenging to achieve with organic compounds alone, allows for a more intricate spatial arrangement of substituents and functional groups. Consequently, these 3D complexes offer improved occupancy of the binding cavity and foster additional interactions within the target protein's binding site, thereby enhancing inhibition potency. Furthermore, the positively charged ruthenium cation in these complexes can engage in supplementary electrostatic interactions with negatively charged amino acids of the protein, further enhancing their inhibitory effects.

One object of the present invention is a compound of the general formula (I) with the following structure:

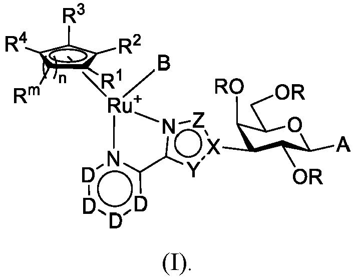

Said compound is a positively charged ruthenium complex, with its positive charge compensated by any pharmaceutically acceptable anion. Suitable anions include, for example, halogens (F⁻, Cl⁻, Br⁻, I⁻), CN⁻, SCN⁻, OCN⁻, PF₆⁻, Zn₂Cl₆²⁻, BF₄⁻, NO₂⁻, NO₃⁻, HSO₄⁻, SO₄²⁻, H₂PO₄⁻, HPO₄²⁻, PO₄³⁻, OH⁻ or CH₃COO⁻.

One of the ligands for the ruthenium cation includes the cyclopentadienyl anion (n=1) or benzene (n=2), which can be either substituted or unsubstituted. These ligands are coordinated to the ruthenium ion via (η⁵-C₅H₅) or (η⁶-C₆H₆) bonds. Accordingly, R¹, R², R³, R⁴, and R^{m} can be independently selected from the group comprising hydrogen and (C₁ to C₆) alkyl groups. Another ligand, B, is selected from the group comprising monovalent negatively charged ligands and neutral ligands. Preferably, B is selected from the group comprising F⁻, Cl⁻, Br⁻, I⁻, CN⁻, SNC⁻, OCN⁻, NO₂⁻, NO₃⁻, HSO₄⁻, OH⁻ or CH₃COO⁻ if the first ligand is a neutral benzene derivative. Alternatively, if the first ligand is a negatively charged cyclopentadienyl derivative, B is preferably selected from the group comprising CO, CO₂ CH₃CN, H₂O, NH₃, monovalent organic N-ligands such as pyridine, or organophosphine ligands such as PPh₃.

The third ligand comprises two aromatic nitrogen heterocycles, which coordinate to the ruthenium cation via their nitrogen atoms. Besides the coordinating nitrogen atoms, these heterocycles may also include other nitrogen (N) atoms or or CH, or carbon. The general formula (I) allows for the following combinations:
X is N;
D and Y are CH, and Z is N.
R is independently H or an acyl of general formula (G) 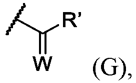
wherein W is O and R' is selected from the group comprising C₁-C₆ alkyl, C₃-C₈ cycloalkyl, and C₆-C₁₂ aryl; preferably R' is selected from phenyl, butyryl, and pivaloyl; more preferably, the substituent R of the general formula (I) is independently H or -C(=O)Ph;
A is selected from the group comprising structures of general formulae (II) and (III). 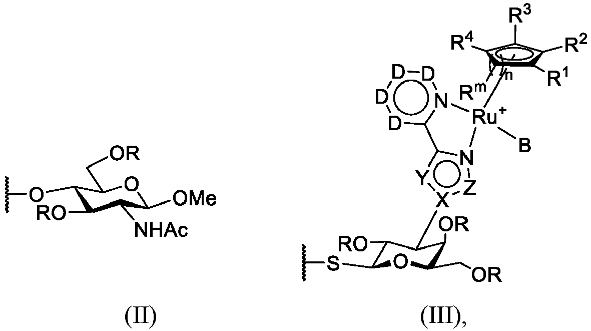
wherein the groups B, X, Z, Y, D, R, R¹, R², R³, R⁴, and R^{m} are as defined above, and *n* is 1 or 2.

As all substituents D are CH, it results in a six-membered pyridine heterocycle.

In one embodiment, R¹, R², R³, R⁴, and R^{m} are independently selected from the group comprising H, -CH₃, and -CH(CH₃)₂.

In one embodiment, all substituents R are H. The complexes wherein R is H are capable of direct inhibition of galectines, especially of galectin-1.

In one embodiment, the substituents R are acyls of general formula (G) as defined above. The complexes wherein R is acyl can be used as prodrugs, as they undergo hydrolysis of acyl groups to OH groups in cytosol, due to the presence of non-specific esterases, thereby forming direct galectin inhibitors *in situ.*

Galectin-1 is present in both intracellular and extracellular environments. Compounds of general formula (I) with free hydroxyl groups (R is H) are designed as inhibitors of extracellular galectin-1 (due to their high polarity, these substances are unable to penetrate the cytoplasmic membrane and enter the cell). Acylated derivatives (R is acyl) are sufficiently lipophilic to be transported through the cytoplasmic membrane into the cytosol of cells. In the cytosol, acyl/thioacyl groups are hydrolyzed and the "active complex" with free hydroxyls is formed, capable of inhibiting intracellular galectin-1. Furthermore, the cytotoxic effect of the ruthenium complex, which may be desirable in certain applications, is also applied inside cells.

The fact that Y of the five-membered heterocycle is CH, and simultaneously X and Z are N, the resulting structure is a five-membered 1,2,3-triazole heterocycle.

In one embodiment, the structural unit (IV) 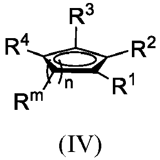 is selected from the group comprising cyclopentadiene, pentamethylcyclopentadiene, benzene, p-cymene, 1,3,5-trimethylbenzene, hexamethylbenzene. Preferably, the structural unit (IV) is *p-*cymene.

In one embodiment, the object of the present invention is a compound of the general formula (Ia) 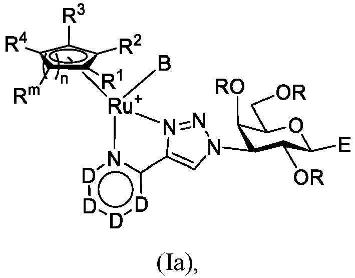 wherein E is the following structure 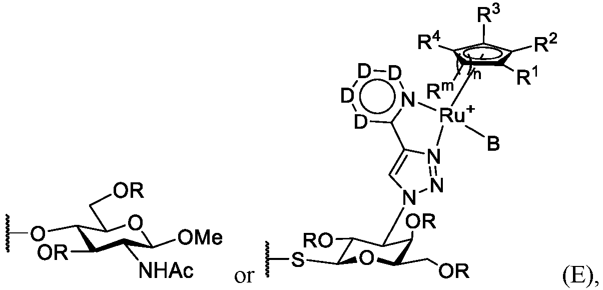 and B, D, R, R¹, R², R³, R⁴, and R^{m} are as defined above.

Preferably, the structural unit (IV) is selected from the group comprising cyclopentadiene, pentamethylcyclopentadiene, benzene, *p*-cymene, 1,3,5-trimethylbenzene, hexamethylbenzene. More preferably, the structural unit (IV) is*p*-cymene.

In the most preferred embodiment, the compound of general formula (I) is selected from the group consisting of: 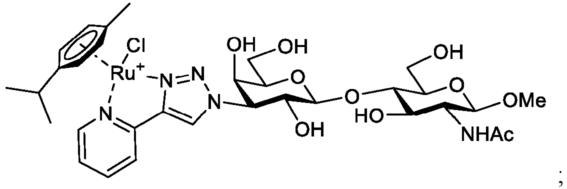 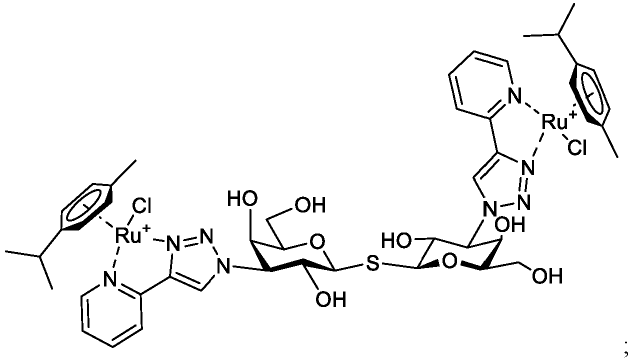 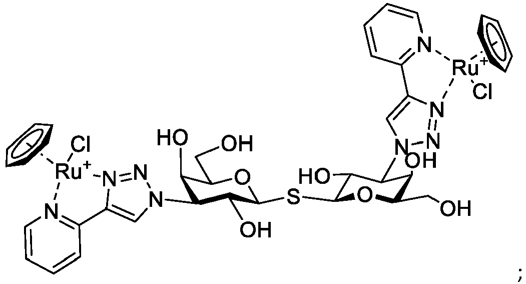 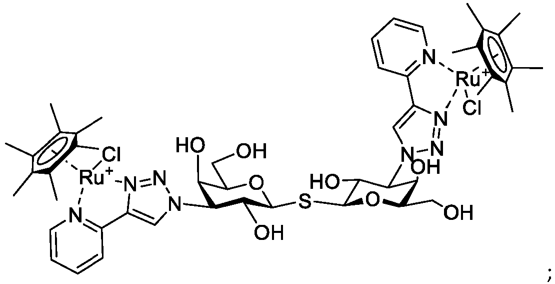 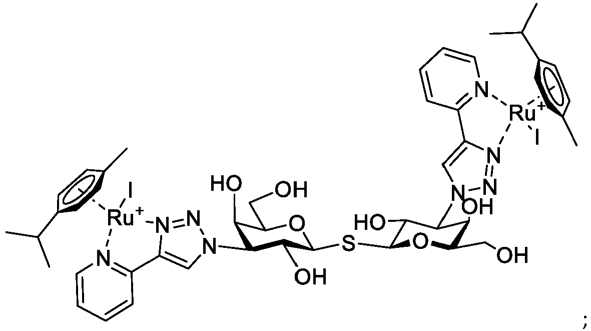 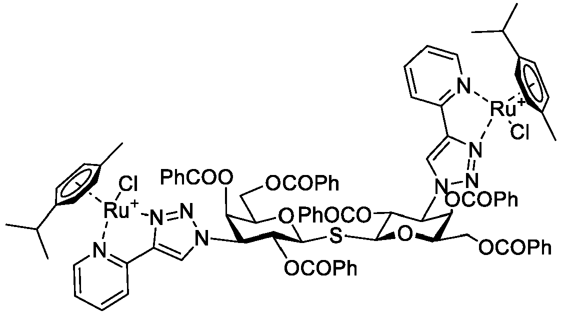 wherein the positive charge of the compound is compensated by an anion selected from the group comprising F⁻, Cl⁻, Br⁻, I⁻, CN⁻, SCN⁻, OCN⁻, PF₆⁻, Zn₂Cl₆²⁻, BF₄⁻, NO₂⁻, NO₃⁻, HSO₄⁻, SO₄²⁻, H₂PO₄⁻, HPO₄²⁻, PO₄³⁻, OH⁻ and CH₃COO⁻, preferably selected from the group comprising Cl⁻, I⁻, BF₄⁻ and PF₆⁻.

Compounds of general formula (I), wherein the five-membered nitrogen heterocycle is a 1,2,3-triazole heterocycle, can be prepared from their azide precursor of general formula (V) 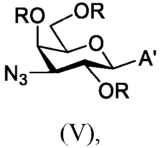 wherein R is as defined above; and A' is 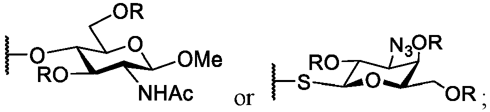 by a reaction with an ethynyl derivative of a six-membered nitrogen heterocycle, such as 2-ethynylpyridine. This reaction involves the Huisgen cycloaddition of the azide group to the triple bond, resulting in the formation of a triazole. Typically, this reaction can be conducted in the presence of copper sulfate and ascorbic acid. Various solvents can be employed, including dimethylformamide, dimethyl sulfoxide, methanol, other alcohols, tetrahydrofuran, ethers, acetone, water, or combinations thereof. The reaction proceeds at the temperature in the range of from 20 to 50 °C, the reaction time ranges from 0.5 to 5 hours, yielding an intermediate product of the general formula (VIa) 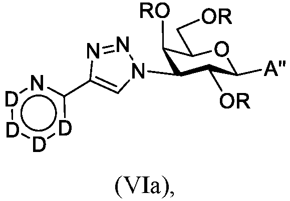 wherein R a D are as defined as above and A" is

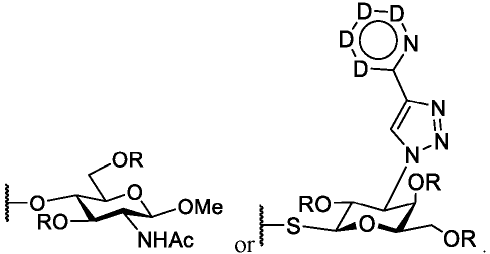

The starting precursor of the general formula (V) is commercially available or can be obtained using standard procedures as described in Mandal, S.; Nilsson, U. J., Tri-isopropylsilyl thioglycosides as masked glycosyl thiol nucleophiles for the synthesis of S-linked glycosides and glyco-conjugates. Organic & Biomolecular Chemistry 2014, 12 (27), 4816-4819; WO 2013/110704 A1; Kurfiřt, M.; Dračínský, M.; Červenková Št'astná, L.; Cuřínová, P.; Hamala, V.; Hovorková, M.; Bojarová, P.; Karban, J., Selectively Deoxyfluorinated N-Acetyllactosamine Analogues as 19F NMR Probes to Study Carbohydrate-Galectin Interactions. Chemistry-A European Journal 2021, 27 (51), 13040-13051.

If R is acyl of the general formula (G), hydrolysis of the acyl group to OH group may be performed as the next step. This step can be carried out, for instance, by basic hydrolysis using sodium methoxide in methanol (e.g., at pH 8 to 10 at room temperature for 10 to 24 hours), resulting in a compound of general formula (VIb) 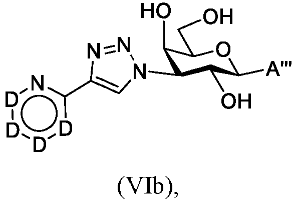 wherein D is as defined above and A‴ is

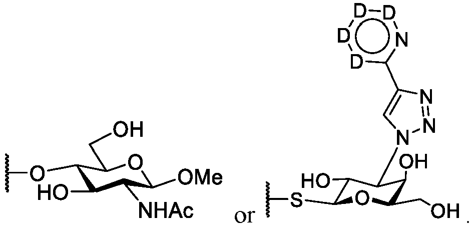

The final step involves the reaction of the compound of the general formula (VIa) or (VIb) with a ruthenium complex of the general formula [RuLB₂]₂, wherein L corresponds to the structural unit (IV) as defined above, and B is as defined above, resulting in a formation of the compound of general formula (I). An example of the ruthenium complex of general formula [RuLB₂]₂ is [Ru(p-cymene)Cl₂]₂. This reaction typically takes place at room temperature in an organic solvent wherein [RuLB₂]₂ is soluble, such as dichloromethane, chloroform, methanol, other alcohols, acetone, water, or mixtures thereof.

Alternatively, the compound of general formula (VIb) may undergo a reaction with the respective acyl chloride of formula R'-C(=O)Cl (e.g. benzoylchloride) before the final step of reaction with ruthenium complex [RuLB₂]₂. This enables to re-introduce the R substituents to the general formula (I). This is especially suitable when R of the general formula (V) is CH₃C(=O)- (acetyl). The compound of general formula (VIb) thus reacts with an acyl chloride of formula R'-C(=O)Cl to form an intermediate of general formula (VIc) 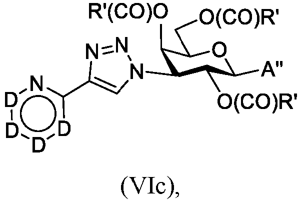
wherein R' and D are as defined above, and
A" is

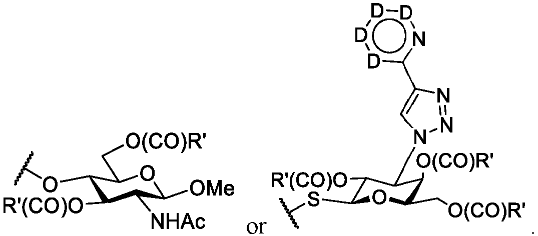

Next step of reaction with ruthenium complex is then performed with said compound of the general formula (VIc) to give the compound of general formula (I).

The advantage of this embodiment is in the simple, low cost and easily obtainable acetyl ester of formula (VIa), which is easily hydrolysed to OH using standard methods (e.g. Zemplén deacylation reaction), followed by the above-mentioned reaction with R'-C(=O)Cl and ruthenium complex to obtain the compound of general formula (I).

In one preferred embodiment, the synthesis of the compound of general formula (I) starts from the azide precursor of general formula (V), wherein the R substituent of is CH₃C(=O)-, and D is as defined above. The step of acyl hydrolysis (e.g. using Zemplén deacylation reaction) may be performed either before the step of Huisgen cycloaddition (the compound of general formula (V) is first hydrolysed followed by Huisgen cycloaddition to form the triazole compound of general formula (VIb)) or following the step of Huisgen cycloaddition (the compound of general formula (V) first undergoes the Huisgen cycloaddition to form the triazole compound of general formula (VIa) followed by deacylation reaction to give the compound of general formula (VIb)).As described above, the compound of general formula (VIb) then either reacts with the respective acyl chloride of formula R'-C(=O)Cl, followed by the reaction with a ruthenium complex of the general formula [RuLB₂]₂, resulting in a formation of the compound of general formula (I), or the compound of general formula (VIb) reacts directly with a ruthenium complex of the general formula [RuLB₂]₂, resulting in a formation of the compound of general formula (I).

In embodiments wherein the anion compensating the positively charged ruthenium complex of the compound of general formula (I) is other than Cl⁻ or I⁻, the reaction of the compound of general formula (VIb) with the ruthenium complex is conducted in the presence of this specific anion. Examples of such anions include MPF₆, MBF₄, MNO₂, MNO₃, MHSO₄, M₂SO₄, MH₂PO₄, M₂HPO₄, M₃PO₄, MOH, CH₃COOM, wherein M in a suitable alkaline metal cation such as Li⁺, Na⁺ or K⁺. The duration of this reaction typically lasts for a few minutes, preferably ranging from 1 to 20 minutes, more preferably from 2 to 15 minutes, and even more preferably from 5 to 10 minutes. Using this method, compounds of the general formula (Ia) can be synthesized 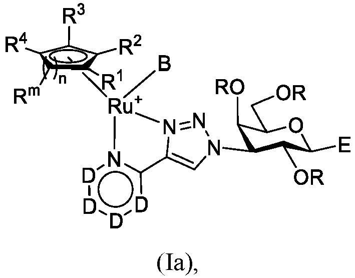 wherein B, D, E, R, R¹, R², R³, R⁴ a R^{m} are as defined above.

Another object of the present invention is the use of compounds of the general formula (I) as defined above. These compounds find application as constituents of analytical probes for assessing the affinity of ligands towards galectins, notably toward galectin-1. Their use in analytical methods either exploits the electroactivity of the ruthenium atom in devising the analytical approach or takes advantage of the ability to covalently bind a fluorophore, such as fluorescein, to the compound of the general formula (I). When equipped with a fluorescently active substituent, the compound of the general formula (I) may serve as a competitive fluorescence probe, enabling the evaluation of the affinity of other substances towards galectins through the fluorescence anisotropy method.

Further use of compounds of the general formula (I) is in medicine, particularly in treatment of diseases associated with the overproduction of galectins, especially of galectin 1, preferably the diseases associated with the overproduction of galectin 1 are selected from the group comprising cancer, fibrotic diseases, and HIV-1 infections.

Further use of compounds of the general formula (I) is for the inhibition of galectins, either *in vivo* or *in vitro.*

The present invention further relates to a pharmaceutical composition, containing at least one compound of the general formula (I) and at least one pharmaceutically acceptable excipient. The pharmaceutically acceptable excipient may be selected from the group comprising carriers, solvents, fillers, binders, disintegrants, coatings, glidants, lubricants, preservatives, flavors, and colorants.

The present invention further relates to a method of treatment, comprising the step of administering a medicament comprising the compound of general formula (I) as defined above, to a subject in need thereof.

The present invention further relates to a method of treatment of diseases associated with the overproduction of galectins, comprising the step of administering a medicament comprising the compound of general formula (I) as defined above, to a subject in need thereof.

The present invention further relates to a method of treatment of cancer, comprising the step of administering a medicament comprising the compound of general formula (I) as defined above, to a subject in need thereof. In one embodiment, the cancer is selected from the group comprising prostate, lung, breast, colon, urinary tract, kidney, lymph nodes, skin, pancreas, upper gastrointestinal tract, thyroid gland, stomach, brain, spinal cord, ovaries, liver, white blood cells, and uterus cancers.

The present invention further relates to a method of treatment of fibrotic diseases, comprising the step of administering a medicament comprising the compound of general formula (I) as defined above, to a subject in need thereof.

The present invention further relates to a method of treatment of HIV-1 infections, comprising the step of administering a medicament comprising the compound of general formula (I) as defined above, to a subject in need thereof.

### Examples

### Synthesis schemes:

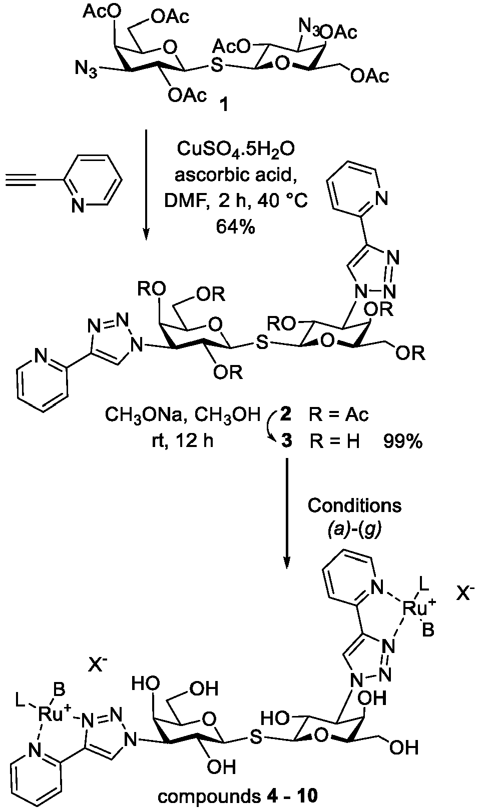

rt = room temperature, conditions (a)-(g) and corresponding reaction yields are shown in the table bellow:

| Compound | L | B | X | Conditions | Yield |
|---|---|---|---|---|---|
| **4** | 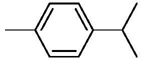 | Cl | Cl | (a) [Ru(*p*-cymen)Cl₂]₂, CH₂Cl₂, CH₃OH, rt, 10 min | 68% |
| **5** | 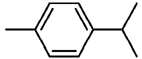 | Cl | BF₄ | (b) [Ru(*p*-cymen)Cl₂]₂, NaBF₄, CH₂Cl₂, CH₃OH, rt, 10 min | 91% |
| **6** | 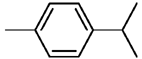 | Cl | PF₆ | (c) [Ru(*p*-cymen)Cl₂]₂, KPF₆, CH₂Cl₂, CH₃OH, rt, 10 min | 58% |
| **7** | 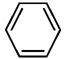 | Cl | PF₆ | (d) [Ru(benzen)Cl₂]₂, KPF₆, CH₂Cl₂, CH₃OH, rt, 10 min | 81% |
| **8** | 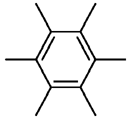 | Cl | PF₆ | (e) [Ru(hexamethylbenzen)Cl₂]₂, KPF₆, CH₂Cl₂, CH₃OH, rt, 10 min | 77% |
| **9** | 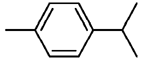 | I | I | (f) [Ru(*p*-cymen)I₂]₂, CH₂Cl₂, CH₃OH, rt, 10 min | 80% |
| **10** | 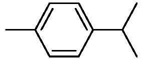 | I | PF₆ | (g) [Ru(*p*-cymen)I₂]₂, KPF₆, CH₂Cl₂, CH₃OH, rt, 10 min | 67% |

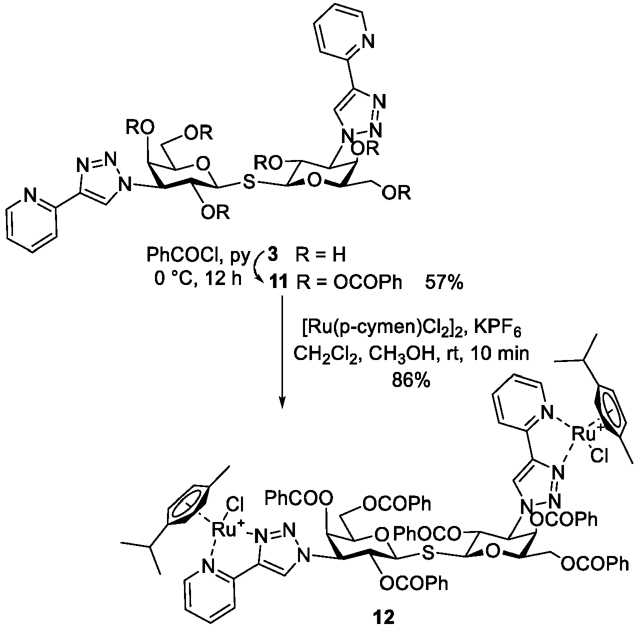

rt = room temperature 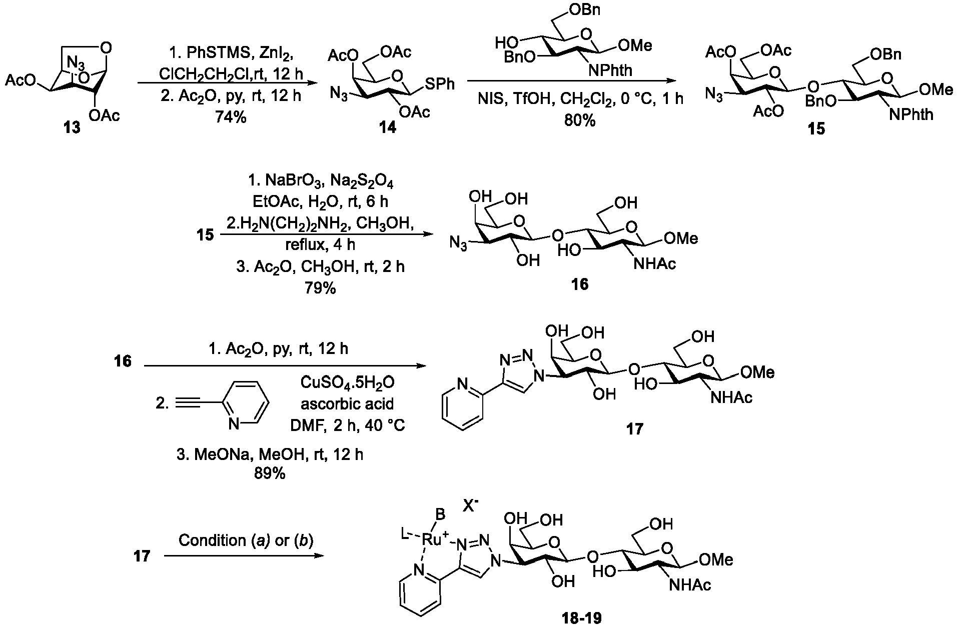
rt = room temperature, conditions (a)-(b) and corresponding reaction yields are shown in the table bellow:

| Compound | L | B | X | Conditions | Yield |
|---|---|---|---|---|---|
| **18** | 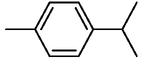 | Cl | Cl | (a) [Ru(*p*-cymen)Cl₂]₂, CH₂Cl₂, CH₃OH, rt, 10 min | 86 % |
| **19** | 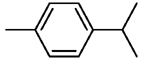 | Cl | PF₆ | (b) [Ru(*p*-cymen)Cl₂]₂, KPF₆, CH₂Cl₂, CH₃OH, rt, 10 min | 73 % |

### Materials and Methods

Chemicals were used as received from suppliers Merck KGaA, Thermo Fisher Scientific, and Fluorochem Ltd. Cell lines HEK-293, A2780, SK-OV-3, MDA-MB-231, MCF-7, and MCF-10A were obtained from the ATCC collection. Dichloromethane was dried by distillation with CaH₂ and stored over 3 Å molecular sieves. Petroleum ether was distilled prior to use. TLC analysis was performed using Sigma-Aldrich TLC Silica gel 60 F254 plates, and compounds were detected using a UV lamp at 254 nm or by staining with an anisaldehyde solution (EtOH, AcOH, H₂SO₄). Column chromatography was conducted on silica gel 60 (70-230 mesh, Material Harvest). Solutions were concentrated using rotary evaporators at temperatures below 45 °C. NMR spectra were recorded on a Bruker Avance 400 NMR spectrometer (¹H at 400.1 MHz, ¹⁹F at 376.4 MHz, ¹³C at 100.6 MHz, ³¹P at 162.0 MHz) at 25 °C. ¹H and ¹³C{¹H} NMR spectra were referenced to the respective solvent peaks (δ/ppm; δH/δC: CDCl₃, 7.26/77.16, MeOH-*d*₄, 3.31/49.00, dmso-*d*₆, 2.50/39.52). ¹⁹F NMR spectra were referenced to an internal standard hexafluorobenzene (-166.62 ppm in MeOH-*d*₄). ³¹P NMR spectra were referenced to an external standard triphenyl phosphate (-17.00 ppm in aceton-*d*₆). Combination of 1D and 2D experiments (¹H-¹H COSY, ¹H-¹³C HSQC, ¹H-¹³C HMBC, ¹H-¹³C HSQC TOCSY) was used for assignment of all hydrogen and carbon signals. HRMS analysis was performed using a Bruker MicrOTOF-QIII instrument, with APCI or ESI ionization in positive or negative mode. EtOAc denotes ethyl acetate, PE petroleum ether, DCM dichloromethane, MeOH methanol, EtOH ethanol, AcOH acetic acid, Ac₂O acetic anhydride, and DMF dimethylformamide. GB139 was prepared according to a known procedure *(*Organic & Biomolecular Chemistry 2014, 12 (27), 4816-4819).

### Example 1: Human galectin-1 and -3 preparation and purification

The coding sequences of human galectin-1 and -3 (NM_002305.4 and NM_002306) were cloned using Gateway recombination technology (Invitrogen) into the pDEST15 vector containing an N-terminal His6-GST tag cleavable by TEV protease (Tobacco Etch Virus nuclear-inclusion-a endopeptidase). Both cloned genes were expressed in *E. coli* BL21 RILP. The bacteria were cultured in 100 mL of LB medium (10 g/L tryptone, 5 g/L yeast extract, 5 g/L NaCl) with ampicillin (100 µg/mL) and chloramphenicol (30 µg/mL) overnight at 37 °C, 220 rpm. The culture was diluted to a total volume of 2.5 liters in LB medium (supplemented with ampicillin and chloramphenicol) to an optical density at A₆₀₀=0.1 and cultured for 2-3 hours. Upon reaching an optical density at A₆₀₀=0.5, gene expression was induced by addition of isopropyl-β-D-thiogalactopyranoside to the culture (final concentration 1 mM). The bacterial culture was further cultured for 4 hours at 30 °C, 140 rpm, and then pelleted by centrifugation (4000 g/4 °C/10 min). Bacterial pellets were resuspended in binding buffer (50 mM Tris-HCl, pH 7.5; 0.15 M NaCl, 0.75% CHAPS). Cell suspensions were subsequently enriched with lysozyme (1 mg/mL), phenylmethylsulfonyl fluoride (1 mM), turbonuclease (1:1000), and MgCl₂ (1 mM), and then sonicated with an ultrasonic sonicator Sonoplus, probe VS 70T, for 15 cycles (10 s sonication, 50 s pause, 35W) at 4 °C. Bacterial lysates were obtained by centrifugation for 30 minutes at 10,000 g/4 °C. His6-GST-tagged proteins were captured on a GSTrap glutathione agarose column (GE Healthcare), eluted with binding buffer enriched with 20 mM glutathione, and subjected to TEV protease cleavage (1 mg of protease per 10 mg of purified protein) overnight at 4 °C. To remove His6-GST and His6-TEV protease, protein mixtures were applied to a HisTrap column (GE Healthcare). Fractions containing purified proteins were collected in a 96-well plate via a fractionator. After concentration, proteins were solubilized in final testing buffers (25 mM Tris-HCl pH 8.0, 300 mM NaCl and 1mM TCEP) using Zeba spin desalting columns that separate molecules with a molecular weight up to 10 kDa (Thermo Fisher Scientific). The purity of all isolated proteins was verified by SDS-PAGE electrophoresis and staining with Coomassie Brilliant Blue. The TEV protease was prepared in the laboratory following the method from Tropea, J. E., Cherry, S., and Waugh, D. S. Expression and purification of soluble His6-tagged TEV protease. Methods Mol. Biol. 2009 498, 297-307).

### Example 2: Synthesis

### 2,2',4,4',6,6'-tetra-O-acetyl-3,3'-diazido-3,3'-dideoxy-1,1'-sulfanediyl-di-β-D-galactopyranoside (1)

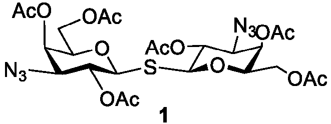

Compound 1 was prepared according to the Mandal, S.; Nilsson, U. J., Tri-isopropylsilyl thioglycosides as masked glycosyl thiol nucleophiles for the synthesis of S-linked glycosides and glyco-conjugates. Organic & Biomolecular Chemistry 2014, 12 (27), 4816-4819.

### 2,2',4,4',6,6'-tetra-O-acetyl-3,3'-dideoxy-3,3'-bis-[4-(pyridin-2-yl)-1H-1,2,3-triazol-1-yl]-1,1'-sulfanediyl-di-β-D-galactopyranoside (2)

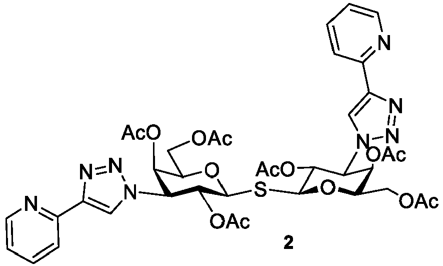

2-Ethynylpyridine (88 µl, 0.87 mmol) was added to a mixture of compound **1** (240 mg, 0.36 mmol), copper(II) sulfate pentahydrate (18 mg, 0.07 mmol), and ascorbic acid (13 mg, 0.07 mmol) in dimethylformamide (3 mL). The reaction mixture was stirred and heated in an oil bath at 40 °C for 2 hours. After cooling the reaction mixture to room temperature, it was diluted with 30 mL of dichloromethane and washed with water (30 mL). The aqueous phase was washed with 2 × 30 mL of dichloromethane. The combined organic phases were dried over anhydrous sodium sulfate. The solvents were evaporated on a rotary evaporator. Column chromatography on 40 g of silica gel in a gradient mixture of EtOAc to EtOAc/CH₃OH 10:1 provided **2** (203 mg, 64%) as a white amorphous solid. *R_{f}* 0.30 (EtOAc). ¹H NMR (dmso-*d*₆, 400 MHz, ¹H-¹H COSY): *δ* 8.61 (ddd, 2H, *J* = 4.8, 1.8, 1.2 Hz, C*H*_{Py}), 8.61 (s, 2H, C*H*_{Tria}), 8.02 (dt, 2H, *J* = 7.9, 1.2 Hz, C*H*_{Py}), 7.90 (ddd, 2H, *J =* 7.9, 7.6, 1.8 Hz, C*H*_{Py}), 7.36 (ddd, 2H, *J =* 7.6, 4.8, 1.2 Hz, C*H*_{Py}), 5.75-5.67 (m, 4H, H-2, H-3), 5.52 (dd, 2H, *J* = 2.8*,* 1.2 Hz, H-4), 5.25 (d, 2H, *J =* 9.6 Hz, H-1), 4.34 (ddd, 2H, *J =* 6.7, 5.9, 1.2 Hz, H-5), 4.14 (dd, 2H, *J =* 11.4, 6.7 Hz, H-6), 4.08 (dd, 2H, *J =* 11.4, 5.9 Hz, H-6'), 2.05, 2.02, 1.87 (3×s, 3 ×6H, *Me*_{Ac})*.* ¹³C{¹H} NMR (dmso-*d*₆, 101 MHz, ¹H-¹³C HSQC, ¹H-¹³C HMBC): *δ* 170.0, 169.3, 169.0 (6×*C*O), 149.7 (2×*C*H_{Py}), 149.5 (2×*C*_{q(Py)}), 147.3 (2×*C*_{q(Ttria)}), 137.3, 123.3 (2×2*C*H_{Py}), 122.9 (2×*C*H_{Tria}), 119.6 (2×*C*H_{Py}), 81.6 (2×C-1), 74.6 (2×C-5), 68.8 (2×C-4), 66.8 (2×C-2), 61.6 (2×C-3), 61.4 (2×C-6), 20.6, 20.3, 20.2 (6×*Me*). HRMS-ESI (m/z): [M + Na]⁺ calculated for C₃₈H₄₂N₈O₁₄SNa, 889.2433; found 889.2437.

### 3,3'-dideoxy-3,3'-bis-[4-(pyridin-2-yl)-1H-1,2,3-triazol-1-yl]-1,1'-sulfanediyl-di-β-D-galactopyranoside (3)

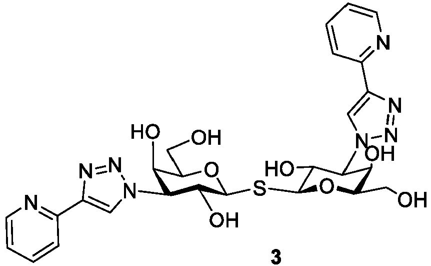

A 0.1 M solution of sodium methanolate in methanol was added dropwise to a solution of **2** (180 mg, 0.21 mmol) in a mixture of methanol (10 mL) and dichloromethane (10 mL) until reaching pH = 9. The reaction mixture was stirred overnight at room temperature. Neutralization with acidic ion exchange resin DOVEX50W, filtration, and solvent evaporation yielded **3** (126 mg, 99%) as a white amorphous solid. *R_{f}* 0.10 (CH₂Cl₂/CH₃OH 7:1). ¹HNMR (dmso-*d*₆, 400 MHz, ¹H-¹H COSY): *δ* 8.60 (dt, 2H, *J =* 4.8, 1.5 Hz, C*H*_{Py}), 8.47 (s, 2H, C*H*_{Tria}), 8.04 (dt, 2H, *J =* 8.0, 1.2 Hz, C*H*_{Py}), 7.90 (ddd, 2H, *J =* 8.0, 7.6, 1.5 Hz, C*H*_{Py}), 7.34 (ddd, 2H, *J =* 7.6, 4.8, 1.2 Hz, C*H*_{Py}), 5.53 (d, 2H, *J =* 6.9 Hz, OH-2), 5.32 (d, 2H, *J* = 7.4 Hz, OH-4), 4.96 (d, 2H, *J =* 9.5 Hz, H-1), 4.92 (dd, 2H, *J =* 10.6, 3.1 Hz, H-3), 4.77 (br s, 2H, O*H*-6), 4.19 (ddd, 2H, *J =* 10.6, 9.5, 6.9 Hz, H-2), 3.99 (dd, 2H, *J =* 7.4, 3.1 Hz, H-4), 3.74 (t, 1H, *J* = 6.4 Hz, H-5), 3.56-3.54 (m, 4H, H-6, H-6'). ¹³C{¹H} NMR (MeOH-*d*₄, 101 MHz, ¹H-¹³C HSQC, ¹H-¹³C HMBC): *δ* 150.2 (2×*C*_{q(Py)}), 149.6 (2×*C*H_{Py}), 146.6 (2×*C*_{q(Tria)}), 137.2, 122.8 (2×2*C*H_{Py}), 122.5 (2×*C*H_{Tria}), 119.3 (2×*C*H_{Py}), 83.5 (2×C-1), 79.3 (2×C-5), 67.6 (2×C-4), 67.0 (2×C-3), 66.9 (2×C-2), 60.1 (2×C-6). HRMS-ESI (m/z): [M + Na]⁺ calculated for C₂₆H₃₀N₈O₈SNa, 637.1800; found 637.1812.

### Ruthenium complex with p-cymene and Cl ligands and Cl⁻ counterions (4)

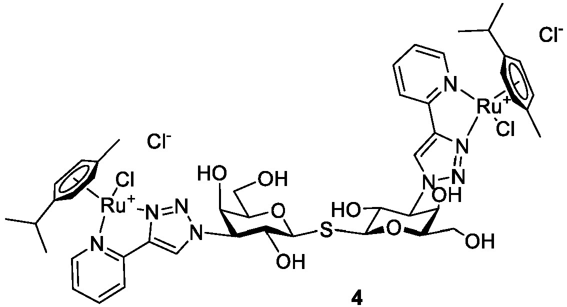

A dark red solution of dichloro(p-cymene)ruthenium dimer (39 mg, 0.06 mmol) in dichloromethane (1 mL) was added to a suspension of **3** (39 mg, 0.06 mmol) in a mixture of methanol/dichloromethane 1:1 (2 mL). A change in color from red to yellow occurred and the starting material completely dissolved. The mixture was stirred at room temperature for 10 minutes. The solvents were evaporated using a rotary vacuum evaporator, the crude mixture was dissolved in a minimal amount of methanol, and the product **4** precipitated by a slow addition of methyl(*tert*-butyl)ether as a yellow solid (52 mg, 67%) mixture of 4 diastereomers in a 2:1:1:1 ratio. To obtain an analytical sample free from traces of residual solvents, the product was dissolved in a small amount of methanol and distilled three times with chloroform. *R_{f}* 0.05 (CH₂Cl₂/CH₃OH 1:1). ¹H NMR (MeOH-*d*₄, 400 MHz, ¹H-¹H COSY): *δ* 9.42-9.41 (m, 4×2H, C*H*_{Py}), 9.15 (s, 2H, C*H*_{Tria}), 9.13 (2×s, 2×2H, C*H*_{Tria}), 9.12 (s, 2H, C*H*_{Tria}), 8.14-8.09 (m, 4×4H, C*H*_{Py}), 7.67-7.64 (m, 4×2H, C*H*_{Py}), 6.14-6.12, 6.08-6.03, 5.94-5.90, 5.85-5.81 (4×m, 4×4×2H, C*H*_{*p-*cym}), 5.23, 5.22 (2×dd, 2×2H, *J =* 10.6, 2.9 Hz, H-3), 5.17 (dd, 2×2H, *J =* 10.6, 2.9 Hz, H-3), 5.02 (4×d, 4×2H, *J =* 9.6 Hz, H-1), 4.76-4.66 (m, 4×2H, H-2), 4.32, 4.31 (2×d, 2×2H, *J =* 2.9 Hz, H-4), 4.27 (2×d, 2×2H, *J =* 2.9 Hz, H-4), 3.97-3.92 (m, 4×2H, H-5), 3.88-3.72 (m, 4×4H, H-6, H-6'), 2.76 (hept, 2H, *J =* 6.8 Hz, *CH*_{*i-*Pr}), 2.24 (s, 2×6H, *Me*_{*p*-cym}), 2.22 (2×s, 2×6H, *Me*_{*p*-cym}), 1.17, 1.16 (2×d, 4×6H, *J* = 6.8 Hz, *Me*_{*i-*Pr})*,* 1.11, 1.07 (2×d, 4×6H, *J* = 6.9 Hz, *Me*_{*i-*Pr})*.* ¹³C{¹H} NMR (MeOH-*d*₄, 101 MHz, ¹H-¹³C HSQC, ¹H-¹³C HMBC, ¹H-¹³C HSQC): *δ* 156.78, 156.75, 156.7 (4×2C*H*_{Py}), 150.0, 149.93, 149.91 (4×2*C*_{q(Py)}), 147.73, 147.72, 147.6 (4×2*C*_{q(Tria)}), 141.5, 141.42, 141.38, 127.39, 127.37, 127.3 (4×4*C*H_{Py}), 126.1, 126.0, 125.73, 125.65 (4×2*C*H_{Tria}), 123.49, 123.46, 123.37, 123.3 (4×2*C*H_{Py}), 106.51, 106.48, 106.46 (4×2*C*_{q}CH_{*i*-Pr}), 103.9, 103.8, 103.7 (4×2*C*_{q}Me), 87.43, 87.39, 87.3 (4×2*C*H_{*p*-cym}), 86.33, 86.27, 86.2 (4×2C-1), 86.11, 86.05, 86.03, 85.9 (4×2*C*H_{*p*-cym}), 85.6, 85.4 (4×2*C*H_{*p*-cym}), 84.8, 84.7, 84.6 (4×2*C*H_{*p*-cym}), 81.12, 81.06, 81.0 (4×2C-5), 71.13, 71.08, 70.9, 70.8 (4×2C-3), 69.61, 69.58, 69.4 (4×2C-4), 68.5, 68.4, 68.24, 68.20 (4×2C-2), 62.51, 62.48 (4×2C-6), 32.33, 32.27 (4×2CH_{*i*-Pr}), 22.7, 22.63, 22.61 (4×2*Me*_{*i*-Pr}), 20.0, 21.8 (4×2*Me*_{*i*-Pr}), 18.80, 18.78, 18.75 (4×2*Me*_{*p*-cym}). HRMS-APCI (m/z): [M - Cl₂]²⁺ calculated for C₄₆H₅₈Cl₂N₈O₈Ru₂S, 578.0781; found 578.0781.

### Ruthenium complex with p-cymene and Cl ligands and BF₄⁻ counterions (5)

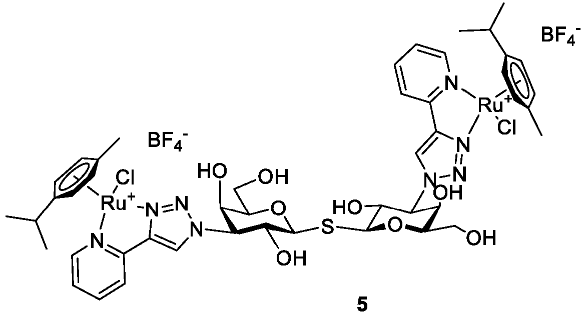

A dark red solution of dichloro(*p*-cymene)ruthenium dimer (24 mg, 0.04 mmol) in dichloromethane (1 mL) was added to a suspension of **3** (24 mg, 0.04 mmol) and NaBF₄ (9 mg, 0.08 mmol) in a mixture of methanol/dichloromethane 1:1 (2 mL). A change in color from red to yellow occurred, the starting material completely dissolved and NaCl precipitated. The mixture was stirred at room temperature for 10 minutes and filtered. The solvents were evaporated using a rotary vacuum evaporator, the crude mixture was dissolved in a minimal amount of methanol, and the product **5** precipitated by a slow addition of methyl *tert*-butyl ether as a yellow solid (47 mg, 91%) mixture of 4 diastereomers in a 5:2:2:1 ratio. To obtain an analytical sample free from traces of residual solvents, the product was dissolved in a small amount of methanol and distilled three times with chloroform. *R_{f}* 0.05 (CH₂Cl₂/CH₃OH 1:1). ¹H NMR (MeOH-*d*₄, 400 MHz, ¹H-¹H COSY): *δ* 9.43-9.41 (m, 4×2H, C*H*_{Py}), 9.16, 9.14, 9.13, 9.12 (4×s, 4×2H, C*H*_{Tria}), 8.18-8.10 (m, 4×4H, C*H*_{Py}), 7.67-7.64 (m, 4×2H, C*H*_{Py}), 6.14-6.12, 6.08-6.03, 5.94-5.91, 5.86-5.82 (4×m, 4×4×2H, C*H*_{*p*-cym}), 5.23 (2×dd, 2×2H, *J =* 10.6, 2.9 Hz, H-3), 5.22, 5.18 (2×dd, 2×2H, *J =* 10.6, 2.9 Hz, H-3), 5.03 (d, 2×2H, *J =* 9.5 Hz, H-1), 5.01, 5.00 (2×d, 2×2H, *J* = 9.5 Hz, H-1), 4.77, 4.73, 4.72, 4.71 (4×dd, 4×2H, *J =* 10.6, 9.5 Hz, H-2), 4.33, 4.32, 4.28, 4.27 (4×d, 4×2H, *J =* 2.9 Hz, H-4), 3.98-3.93 (m, 4×2H, H-5), 3.88-3.73 (m, 4×4H, H-6a, H-6b), 2.82-2.70 (m, 4×2H, C*H*_{*i*-Pr}), 2.24, 2.22. 2.21 (3×s, 4×6H, *Me*_{*p*-cym}), 1.17, 1.16, 1.12, 1.11, 1.07 (5×d, 4×12H, *J =* 6.9 Hz, *Me*_{*i-*Pr})*.* ¹³C{¹H} NMR (MeOH-*d*₄, 101 MHz, ¹H-¹³C HSQC, ¹H-¹³C HMBC, ¹H-¹³C HSQC): *δ* 156.8, 156.74, 156.70 (4×2C*H*_{Py}), 150.0, 149.9 (4×2*C*_{q(Py)}), 147.73, 147.71, 147.6 (4×2*C*_{q(Tria)}), 141.5, 141.42, 141.36 (4×2C*H*_{Py}), 127.4, 127.3 (4×2C*H*_{Py}), 126.02, 125.97, 125.74, 125.69 (4×2*C*H_{Tria}), 123.54, 123.50, 123.4 (4×2C*H*_{Py}), 106.51, 108.49, 108.47, 106.45 (4×2*C*_{q}CH_{*i*-Pr}), 103.9, 103.70, 103.68, 103.6 (4×2*C*_{q}Me), 87.5, 87.4, 87.3 (4×2*C*H_{*p*-cym}), 86.30, 86.27, 86.2 (4×2C-1), 86.03, 86.00, 85.9, 85.6, 85.4, 84.8, 84.68, 84.65 (4×6*C*H_{*p*-cym}), 81.12, 81.07, 81.05 (4×2C-5), 71.1, 71.0, 70.8 (4×2C-3), 69.6, 69.5, 69.4 (4×2C-4), 68.4, 68.20, 68.16 (4×2C-2), 62.5 (4×2C-6), 33.32, 33.26 (4×2*C*H_{*i*-Pr}), 22.7, 22.6, 22.0, 21.8 (4×2*Me*_{*i*-Pr}), 18.80, 18.78, 18.76 (4×2*Me*_{*p*-cym}). ¹⁹F NMR (MeOH-*d*₄, 376 MHz): *δ* -155.45 (br s, ¹¹BF₄), -155.50 (br s, ¹⁰BF₄). HRMS-APCI (m/z): [M - 2 BF₄]²⁺ calculated for C₄₆H₅₈Cl₂N₈O₈Ru₂S, 578.0781; found 578.0784.

### Ruthenium complex with p-cymene and Cl ligands and PF6 counterions (6)

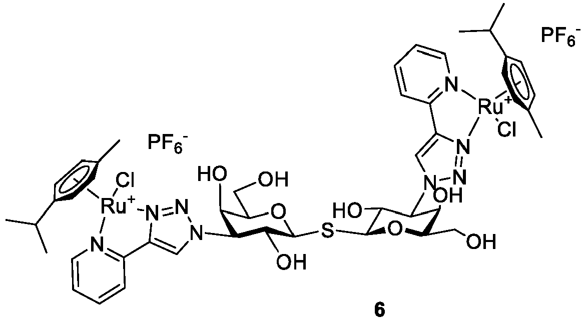

A dark red solution of dichloro(*p*-cymene)ruthenium dimer (33 mg, 0.05 mmol) in dichloromethane (1 mL) was added to a suspension of **3** (33 mg, 0.05 mmol) and KPF₆ (20 mg, 0.10 mmol) in a mixture of methanol/dichloromethane 1:1 (2 mL). A change in color from red to yellow occurred, the starting material completely dissolved and KCl precipitated. The mixture was stirred at room temperature for 10 minutes and filtered. The solvents were evaporated using a rotary vacuum evaporator, the crude mixture was dissolved in a minimal amount of methanol, and the product **6** precipitated by a slow addition of methyl tert-butyl ether as a yellow solid (45 mg, 58%) mixture of 4 diastereomers in a 5:2:2:1 ratio. To obtain an analytical sample free from traces of residual solvents, the product was dissolved in a small amount of methanol and distilled three times with chloroform. *R_{f}* 0.05 (CH₂Cl₂/CH₃OH 1:1). ¹H NMR (MeOH-*d*₄, 400 MHz, ¹H-¹H COSY): *δ* 9.42-9.41 (m, 4×2H, C*H*_{Py}), 9.16, 9.15 (2×s, 2×2H, C*H*_{Tria}), 9.13 (2×s, 2×2H, C*H*_{Tria}), 8.19-8.13 (m, 4×4H, C*H*_{Py}), 7.67-7.64 (m, 4×2H, C*H*_{Py}), 6.14-6.13, 6.08-6.03, 5.95-5.91, 5.87-5.82 (4×m, 4×8H, C*H*_{*p-*cym}), 5.28, 5.25, 5.22 (3×dd, 4×2H, *J* = 10.7, 2.9 Hz, H-3), 5.05, 5.03, 5.02, 5.01 (4×d, 4×2H, *J* = 9.6 Hz, H-1), 4.79-4.69 (m, 4×2H, H-2), 4.35, 4.28 (2×d, 4×2H, *J* = 2.9 Hz, H-4), 3.99-3.96 (m, 4×2H, H-5), 3.86-3.74 (m, 4×4H, H-6a, H-6b), 2.81-2.70 (m, 4×2H, CH_{*i*-Pr}), 2.23, 2.20 (2×s, 4×6H,*Me*_{*p-*cym}), 1.17, 1.16, 1.11, 1.07 (4×d, 4× 12H, *J* = 7.0 Hz, *Me*_{*i*-Pr}). ¹³C{¹H} NMR (MeOH-*d*₄, 101 MHz, ¹H-¹³C HSQC, ¹H-¹³C HMBC): *δ* 156.79, 156.76, 156.72 (4×2C*H*_{Py}), 150.0, 149.91, 149.89 (4×2*C*_{q(Py)}), 147.74, 147.72, 147.6 (4×2*C*_{q(Tria)}), 141.5 141.41,141.37 (4×2C*H*_{Py}), 127.40, 127.35 (4×2C*H*_{Py}), 126.0, 125.9, 125.7, 125.6 (4×2*C*H_{Tria}), 123.49, 123.45, 123.3 (4×2*C*H_{Py}), 106.50, 106.49, 106.47 (4×2*C*_{q}CH_{*i*-Pr}), 103.91, 103.90, 103.7 (4×2*C*_{q}Me), 87.5, 87.3 (4×2*C*H_{*p*-cym}), 86.33, 86.31, 86.25 (4×2C-1), 86.04, 86.03, 85.9 (4×2*C*H_{*p*-cym}), 85.6, 85.4 (4×2*C*H_{*p*-cym}), 84.8, 84.6 (4×2*C*H_{*p*-cym}), 81.13, 81.08, 81.07, 81.06 (4×2C-5), 71.1, 70.9, 70.8 (4×2C-3), 69.63, 69.60, 69.4 (4×2C-4), 68.41, 68.38, 68.23, 68.20 (4×2C-2), 62.5 (4×2C-6), 32.32, 32.26 (4×2*C*H_{*i-*Pr}), 22.64, 22.62, 22.60, 22.0, 21.9, 21.8 (4×2*Me*_{*i*-Pr}), 18.77, 18.75, 18.7 (4×2*Me*_{*p*-cym}). ³¹P{¹H} NMR (MeOH-*d*₄, 162 MHz): *δ* -161.37 (hept, ¹*J*_{(P-F)} = 708.4 Hz). ¹⁹F NMR (MeOH-*d*₄, 376 MHz): *δ* -75.31 (d, ¹*J*_{(F-P)} = 708.4 Hz).

¹H NMR (D₂O, 400 MHz, ¹H-¹H COSY): *δ* 9.40 (dd, 4×2H, *J =* 5.7, 1.4 Hz, C*H*_{Py}), 9.06 (s, 2×2H, C*H*_{Tria}), 9.03 (2×s, 2×2H, C*H*_{Tria}), 8.18 (ddd, 4×2H, *J* = 7.7, 7.4, 1.4 Hz, C*H*_{Py}), 8.09 (dd, 4×2H, *J* = 7.7, 1.5 Hz, C*H*_{Py}), 7.68 (ddd, 4×2H, *J* = 7.4, 5.7, 1.5 Hz, C*H*_{Py}), 6.22, 6.19 (2×d, 2×4H, *J* = 6.4 Hz, C*H*_{*p-*cym}), 6.11, 6.10 (2×d, 2×2H, *J =* 6.2 Hz, C*H*_{*p*-cym}), 6.07 (d, 2×2H, *J =* 6.2 Hz, C*H*_{*p*-cym}), 5.99, 5.95 (2×d, 2×4H, *J =* 6.2 Hz, C*H*_{*p-*cym}), 5.87 (2×d, 2×2H, *J =* 6.4 Hz, C*H*_{*p*-cym}), 5.85 (d, 2×2H, *J* = 6.4 Hz, C*H*_{*p-*cym}), 5.31, 5.28 (2×dd, 2×4H, *J =* 10.7, 3.0 Hz, H-3), 5.24 (d, 2H, *J =* 9.8 Hz, H-1), 5.23 (2×d, 2×2H, *J =* 9.8 Hz, H-1), 5.22 (d, 2H, *J* = 9.8 Hz, H-1), 4.60, 4.59, 4.51, 4.50 (4×dd, 4×2H, *J =* 10.7, 9.8 Hz, H-2), 4.41, 4.40 (2×d, 2×4H, *J* = 3.0 Hz, H-4), 4.14-4.10 (m, 4×2H, H-5), 3.93-3.87 (m, 4×2H, H-6a), 3.84-3.79 (m, 4×2H, H-6b), 2.73-2.59 (m, 4×2H, C*H*_{*i*-Pr}), 2.22, 2.21 (2×s, 4×6H, *Me*_{*p*-cym}), 1.09, 1.08, 1.03, 0.99 (4×d, 4×12H, *J =* 6.9 Hz, *Me*_{*i-*Pr})*.* ¹³C{¹H} NMR (D₂O, 101 MHz, ¹H-¹³C HSQC, ¹H-¹³C HMBC): *δ* 155.8, 155.7 (4×2C*H*_{Py}), 148.5 (4×2*C*_{q(Py)}), 147.5, 147.4 (4×2*C*_{q(Tria)}), 141.2 (4×2C*H*_{Py}), 127.3 (4×2*C*H_{Py}), 125.7, 125.2 (4×2*C*H_{Tria}), 123.5 (4×2C*H*_{Py}), 105.3, 105.2 (4×2*C*_{q}CH_{*i*-Pr}), 103.9, 103.72, 103.70 (4×2*C*_{q}Me), 86.7, 86.6 (4×2*C*H_{*p*-cym}), 85.84, 85.81, 85.6 (4×2*C*H_{*p*-cym}), 84.83, 84.80 (4×2C-1), 84.9, 84.64, 854.62 (4×2*C*H_{*p*-cym}), 83.78, 83.76 (4×2*C*H_{*p*-cym}), 80.2, 82.1 (4×2C-5), 69.5, 69.3 (4×2C-3), 68.6, 68.4 (4×2C-4), 67.5, 67.4, 67.3 (4×2C-2), 61.63, 61.61, 61.60 (4×2C-6), 31.4, 31.3, 31.2 (4×2*C*H_{*i*-Pr}), 21.92, 21.90, 21.89, 21.4, 21.3 (4×2*Me*_{*i*-Pr}), 18.6 (4×2*Me*_{*p-*cym}). ³¹P{¹H} NMR (D₂O, 162 MHz): *δ* -161.97 (hept, ¹*J*_{(P-F)} = 708.9 Hz). ¹⁹F NMR (D₂O, 376 MHz): *δ -* 73.32 (d, ¹*J*_{(F-P)} = 708.9 Hz). HRMS-APCI (m/z): [M - 2 PF₆]²⁺ calculated for C₄₆H₅₈Cl₂N₈O₈Ru₂S, 578.0781; found 578.0787.

### Ruthenium complex with benzene and Cl ligands and PF₆⁻ counterions (7)

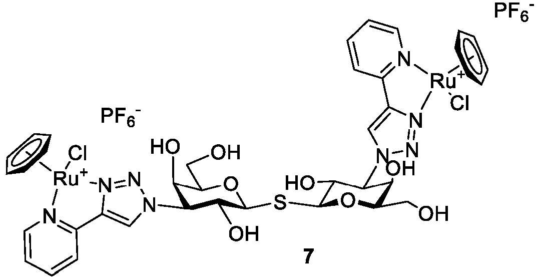

A dark red solution of dichloro(benzene)ruthenium dimer (23 mg, 0.05 mmol) in dichloromethane (1 mL) was added to a suspension of 3 (28 mg, 0.05 mmol) and KPF₆ (17 mg, 0.09 mmol) in a mixture of methanol/dichloromethane 1:1 (2 mL). A change in color from red to yellow occurred, the starting material completely dissolved and KCl precipitated. The mixture was stirred at room temperature for 10 minutes and filtered. The solvents were evaporated using a rotary vacuum evaporator, the crude mixture was dissolved in a minimal amount of methanol, and the product 7 precipitated by a slow addition of methyl tert-butyl ether as a yellow solid (49 mg, 81%) mixture of 4 diastereomers. To obtain an analytical sample free from traces of residual solvents, the product was dissolved in a small amount of methanol and distilled three times with chloroform. *R_{f}* 0.05 (CH₂Cl₂/CH₃OH 1:1). ¹H NMR (MeOH-*d*₄, 400 MHz, ¹H-¹H COSY): *δ* 9.59-9.57 (m, 4×2H, C*H*_{Py}), 9.42, 9.40 (2×s, 4×2H, C*H*_{Tria}), 8.30-8.22 (m, 4×4H, C*H*_{Py}), 7.69-7.66 (m, 4×2H, C*H*_{Py}), 6.19, 6.18 (2×s, 4×10H, C*H_{Ph}*), 5.80-5.77 (m, 4×2H, OH-2), 5.72-5.65 (m, 4×2H, OH-4), 5.23, 5.20 (2×dd, 4×2H, *J* = 10.6, 2.9 Hz, H-3), 5.04, 5.03 (2×d, 4×2H, *J* = 9.5 Hz, H-1), 4.92, 4.85 (2×t, 4×2H, *J* = 6.0 Hz, OH-6), 4.30-4.20 (m, 10H, H-2, H-4), 4.12-4.06 (m, 3×2H, H-4), 3.82-3.79 (m, 4×2H, H-5), 3.61-3.57 (m, 4×4H, H-6a, H-6b). ¹³C{¹H} NMR (MeOH-*d*₄, 101 MHz, ¹H-¹³C HSQC, ¹H-¹³C HMBC, ¹H-¹³C HSQC): *δ 156.0* (4×2C*H*_{Py}), 148.28, 148.25 (4×2*C*_{q(Py)}), 145.7, 145.5 (4×2*C*_{q(Tria)}), 141.3 (4×2C*H*_{Py}), 125.7 (4×2C*H*_{Py}), 125.5, 125.4 (4×2*C*H_{Tria}), 122.1, 122.0 (4×2C*H*_{Py}), 85.6 (4×10*C*H*_{Ph}*), 83.3, 83.21, 83.16, 83.1 (4×2C-1), 79.0, 78.8 (4×2C-5), 69.1, 79.0 (4×2C-3), 67.1 (4×2C-4), 66.8, 66.6 (4×2C-2), 59.9, 59.6 (4×2C-6). ³¹P{¹H} NMR (MeOH-*d*₄, 162 MHz): *δ* -158.91 (hept, ¹*J*_{(P-F)} = 711.4 Hz). ¹⁹F NMR (MeOH-*d*₄, 376 MHz): *δ* -71.43 (d, ¹*J*_{(F-P)} = 711.4 Hz). HRMS-APCI (m/z): [M - 2 PF₆]²⁺ calculated for C₃₈H₄₂Cl₂N₈O₈Ru₂S, 522.0154; found 522.0158.

### Ruthenium complex with hexamethylbenzene and Cl ligands and PF₆⁻ counterions (8)

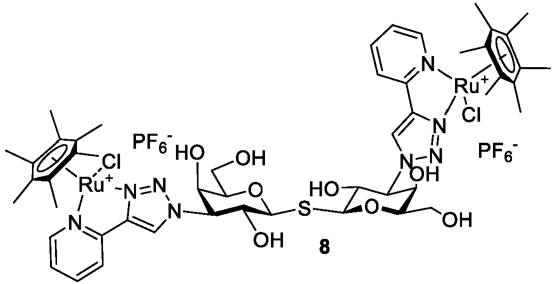

A dark red solution of dichloro(benzene)ruthenium dimer (33 mg, 0.05 mmol) in dichloromethane (1 mL) was added to a suspension of 3 (30 mg, 0.05 mmol) and KPF₆ (18 mg, 0.09 mmol) in a mixture of methanol/dichloromethane 1:1 (2 mL). A change in color from red to yellow occurred, the starting material completely dissolved and KCl precipitated. The mixture was stirred at room temperature for 10 minutes and filtered. The solvents were evaporated using a rotary vacuum evaporator, the crude mixture was dissolved in a minimal amount of methanol, and the product 8 precipitated by a slow addition of methyl *tert*-butyl ether as a yellow solid (56 mg, 77%) mixture of 4 diastereomers. To obtain an analytical sample free from traces of residual solvents, the product was dissolved in a small amount of methanol and distilled 3 times with chloroform. *R_{f}* 0.05 (CH₂Cl₂/CH₃OH 1:1). ¹H NMR (dmso-*d*₆, 400 MHz, ¹H-¹H COSY): *δ* 9.43, 9.42, 9.41 (3×s, 4×2H, C*H*_{Tria}), 8.91-8.89 (m, 4×2H, C*H*_{Py}), 8.30-8.18 (m, 4×4H, C*H*_{Py}), 7.71-7.67 (m, 4×2H, C*H*_{Py}), 5.80-5.72 (m, 4×4H, *OH-2, OH-4),* 5.25 (dd, 4×2H, *J* = 10.6, 3.2 Hz, H-3), 5.08, 5.04 (3×d, 4×2H, *J* = 9.5 Hz, H-1), 4.99, 4.96, 4.85, 4.88 (4×t, 4×2H, *J* = 6.1 Hz, OH-6), 4.26-4.09 (m, 4×4H, H-2, H-4), 3.84, 3.79 (2×t, 4×2H, *J* = 6.7 Hz, H-5), 3.60-3.58 (m, 4×4H, H-6, H-6'), 2.11, 2.10, 1.7 (3×s, 4×36H, *Me*). ¹³C{¹H} NMR (dmso-*d*₆, 101 MHz, ¹H-¹³C HSQC, ¹H-¹³C HMBC, ¹H-¹³C HSQC TOCSY): *δ* 153.0 (4×2C*H*_{Py}), 148.04, 147.95, 147.94 (4×2*C*_{q(Py)}), 145.5, 145.2 (4×2*C*_{q(Tria)}), 139.7 (4×2C*H*_{Py}), 126.0 (4×2C*H*_{Py}), 125.2 (4×2*C*H_{Tria}), 121.6, 121.5 (4×2C*H*_{Py}), 96.1, 94.8 (4×12*C*_{q}Me), 83.0, 82.92, 82.85 (4×2C-1), 79.1, 78.8 (4×2C-5), 69.0 (4×2C-3), 67.5 (4×2C-2), 67.2, 66.8, 66.5 (4×2C-4), 59.8, 59.6 (4×2C-6), 16.6, 15.4, 15.28, 15.26 (4×12*Me*). ³¹P{¹H} NMR (dmso-*d*₆, 162 MHz): *δ* -161.10 (hept, ¹*J*_{(P-F)} = 711.4 Hz). ¹⁹F NMR (dmso-*d*₆, 376 MHz): *δ* -71.44 (d, ¹*J*_{(F-P)} = 711.4 Hz). HRMS-APCI (m/z): [M - 2 PF₆]²⁺ calculated for C₅₀H₆₆Cl₂N₈O₈Ru₂S 606.2096; found 606.1098.

### Ruthenium complex with p-cymene and I ligands and I counterions (9)

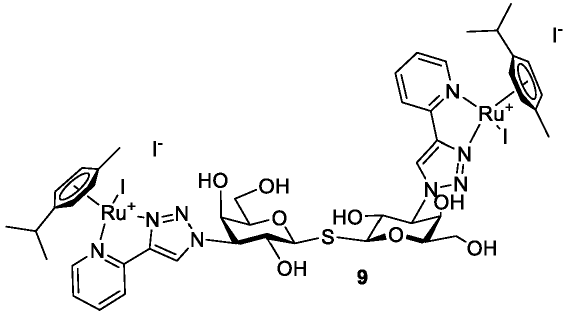

A dark red solution of dichloro(p-cymene)ruthenium dimer (19 mg, 0.02 mmol) in dichloromethane (1 mL) was added to a suspension of **3** (12 mg, 0.02 mmol) in a mixture of methanol/dichloromethane 1:1 (2 mL). A change in color from red to yellow occurred and the starting material completely dissolved. The mixture was stirred at room temperature for 10 minutes. The solvents were evaporated using a rotary vacuum evaporator, the crude mixture was dissolved in a minimal amount of methanol, and the product 9 precipitated by a slow addition of methyl tert-butyl ether as a yellow solid (25 mg, 80%) mixture of 4 diastereomers in a 5:2:2:1 ratio. To obtain an analytical sample free from traces of residual solvents, the product was dissolved in a small amount of methanol and distilled three times with chloroform. *R_{f}* 0.05 (CH₂Cl₂/CH₃OH 1:1). ¹H NMR (MeOH-*d*₄, 400 MHz, ¹H-¹H COSY): *δ* 9.35-9.32 (m, 4×2H, C*H*_{Py}), 9.19, 9.17, 9.15, 9.13 (4×s, 4×2H, C*H*_{Tria}), 8.25-8.08 (m, 4×4H, C*H*_{Py}), 7.60-7.58 (m, 4×2H, C*H*_{Py}), 6.09-5.98, 5.94-5.84 (2×m, 2×4×4H, C*H*_{*p*-cym}), 5.23 (2×dd, 3×4H, *J* = 10.7, 2.9 Hz, H-3), 5.20 (dd, 2H, *J* = 10.7, 2.9 Hz, H-3), 5.02 (d, 2×2H, *J* = 9.6 Hz, H-1), 4.97, 4.96 (2×d, 2×2H, *J* = 9.6 Hz, H-1), 4.81-4.69 (m, 4×2H, H-2), 4.38, 4.36, 4.26, 4.25 (4×d, 4×2H, J= 2.9 Hz, H-4), 3.99-3.92 (m, 4×2H, H-5), 3.84-3.71 (m, 4×4H, H-6, H-6'), 3.84-3.71 (m, 4×2H, C*H*_{*i*-Pr}), 2.39, 2.33. 2.32 (3×s, 4×6H, *Me*_{*p*-cym}), 1.22-1.09 (m, 4×12H, *Me*_{*i*-Pr}). ¹³C{¹H} NMR (MeOH-*d*₄, 101 MHz, ¹H-¹³C HSQC, ¹H-¹³C HMBC): *δ* 158.1, 158.0, 157.9 (4×2C*H*_{Py}), 150.1, 150.0,149.9, 149.8 (4×2*C*_{q(Py)}), 147.58, 147.56, 147.43. 147.41 (4×2*C*_{q(Tria)}), 141.0, 140.94, 140.91, 140.8 (4×2C*H*_{Py}), 127.00, 126.98, 126.96, 126.9 (4×2C*H*_{Py}), 125.8, 125.6 (4×2*C*H_{Tria}), 123.7, 123.3, 123.2 (4×2C*H*_{Py}), 108.5, 108.4, 108.0, 107.9 (4×2*C*_{q}CH_{*i*-Pr}), 102.6 (4×2*C*_{qM}e), 87.4, 86.74, 86.71, 86.69, 86.6, 86.4, 85.77, 85.75, 85.7, 85.6 (4×8*C*H_{*p*-cym}, 4×C-1), 81.2, 81.1, 81.0 (4×C-5), 70.7, 70.6 (4×C-3), 69.4, 69.3 (4×C-4), 68.6, 68.1, 68.0 (4×C-2), 62.6, 62.61, 62.57 (4×C-6), 33.1, 33.0, 32.9 (4×2*C*H_{*i-*Pr}), 23.2, 23.1, 22.9, 22.1, 22.0, 21.9 (4×2*Me*_{*i*-Pr}), 20.1, 19.9 (4×2*Me*_{*p*-cym}). HRMS-APCI (m/z): [M - I₂]²⁺ calculated for C₄₆H₅₈I₂N₈O₈Ru₂S 670.0143; found 670.0135.

### Ruthenium complex with p-cymene and I ligands and PF₆⁻ counterions (10)

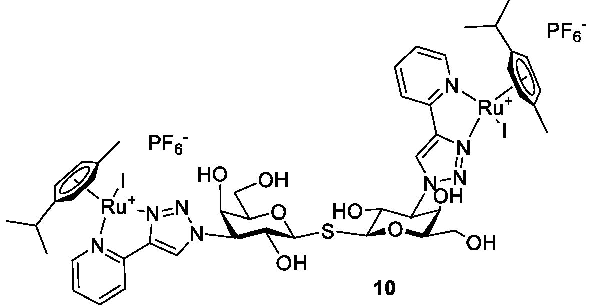

A dark red solution of dichloro(p-cymene)ruthenium dimer (48 mg, 0.05 mmol) in dichloromethane (1 mL) was added to a suspension of 3 (30 mg, 0.05 mmol) and KPF₆ (18 mg, 0.10 mmol) in a mixture of methanol/dichloromethane 1:1 (2 mL). A change in color from red to yellow occurred, the starting material completely dissolved and KCl precipitated. The mixture was stirred at room temperature for 10 minutes and filtered. The solvents were evaporated using a rotary vacuum evaporator, the crude mixture was dissolved in a minimal amount of methanol, and the product **10** precipitated by a slow addition of methyl *tert*-butyl ether as a yellow solid (52 mg, 67%) mixture of 4 diastereomers in a 5:2:2:1 ratio. To obtain an analytical sample free from traces of residual solvents, the product was dissolved in a small amount of methanol and distilled three times with chloroform. *R_{f}* 0.05 (CH₂Cl₂/CH₃OH 1:1). ¹H NMR (MeOH-*d*₄, 400 MHz, ¹H-¹H COSY): *δ* 9.35-9.32 (m, 4×2H, C*H*_{Py}), 9.18, 9.17, 9.14, 9.12 (4×s, 4×2H, C*H*_{Tria}), 8.25-8.08 (m, 4×4H, C*H*_{Py}), 7.61-7.56 (m, 4×2H, C*H*_{Py}), 6.09-6.02, 6.02-5.98, 5.94-5.87, 5.85-5.84 (4×m, 4×4×2H, C*H*_{*p*-cym}), 5.24, 5.23, 5.21 (3×dd, 4×2H, *J* = 10.7, 2.9 Hz, H-3), 5.02, 4.97, 4.96, 4.95 (4×d, 4×2H, *J* = 9.5 Hz, H-1), 4.84, 4.82, 4.72 (3×dd, 4×2H, *J* = 10.7, 9.5 Hz, H-2), 4.39, 4.38, 4.26, 4.25 (4×d, 4×2H, *J* = 2.9 Hz, H-4), 4.00-3.93 (m, 4×2H, H-5), 3.84-3.71 (m, 4×4H, H-6, H-6'), 3.06-2.90 (m, 4×2H, C*H*_{*i*-Pr}), 2.39, 2.33. 2.31 (3×s, 4×6H, *Me*_{*p*-cym}), 1.21, 1.20, 1.19, 1.10 (4×d, 4×12H, *J* = 6.7 Hz, *Me*_{*i*-Pr}). ¹³C{¹H} NMR (MeOH-*d*₄, 101 MHz, ¹H-¹³C HSQC, ¹H-¹³C HMBC, ¹H-¹³C HSQC TOCSY): *δ* 158.1, 158.0, 157.91, 157.90 (4×2C*H*_{Py}), 150.0, 149.9,149.8, 149.7 (4×2*C*_{q(Py)}), 147.6, 147.5, 147.41, 147.38 (4×2*C*_{q(Tria)}), 141.0, 140.9, 140.8 (4×2C*H*_{Py}), 126.98, 126.96, 126.9 (4×2C*H*_{Py}), 125.7, 125.6, 125.5 (4×2*C*H_{Tria}), 123.7, 123.3, 123.2 (4×2C*H*_{Py}), 108.50, 108.45, 107.9, 107.8 (4×2*C*_{q}CH_{*i-P*r}), 102.61, 102.58, 102.55 (4×2*C*_{q}Me), 87.5, 87.3, 86.70, 86.66, 86.6, 86.5, 86.3, 85.8, 85.7, 85.62, 85.56, 85.55 (4×8*C*H_{*p*-cym}, 4×2C-1), 81.2, 81.1, 81.0 (4×2C-5), 70.60, 70.55, 70.5 (4×2C-3), 69.4, 69.33, 69.28 (4×2C-4), 68.53, 68.51, 67.99, 67.95 (4×2C-2), 62.7, 62.60, 62.55 (4×2C-6), 33.04, 33.02, 32.8 (4×2*C*H_{*i*-pr}), 23.2, 23.1, 22.9, 22.0, 21.91, 21.90 (4×2*Me*_{*i*-Pr}), 20.10, 20.09, 19.9, 19.8 (4×2*Me*_{*p*-cym}). ³¹P{¹H} NMR (MeOH-*d*₄, 162 MHz): *δ* -159.24 (hept, ¹*J*_{(P-F)} = 708.4 Hz). ¹⁹F NMR (MeOH-*d*₄, 376 MHz): *δ* -75.66 (d, ¹*J*_{(F-P)} = 708.4 Hz). HRMS-APCI (m/z): [M - 2 PF₆]²⁺ calculated for C₄₆H₅₈I₂N₈O₈Ru₂S 670.0143; found 670.0148.

### 2,2',4,4',6,6'-tetra-O-benzoyl-3,3'-dideoxy-3,3'-bis-[4-(pyridin-2-yl)-1H-1,2,3-triazol-1-yl]-1,1'-sulfanediyl-di-β-D-galactopyranoside (11)

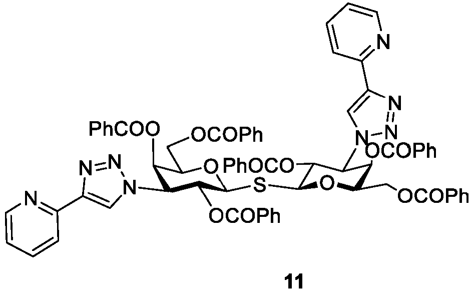

Benzoyl chloride (272 µL, 2.34 mmol) was dropwise added into a solution of 3 (120 mg, 0.20 mmol) in pyridine (4 mL) at 0°C and the reaction mixture was stirred at 0 °C overnight. The reaction mixture was diluted with dichloromethane (30 mL) and washed with water (30 mL). The aqueous phase was extracted two times with dichloromethane (20 mL). The combined organic phases were dried over anhydrous sodium sulfate. The solvents were evaporated on a rotary evaporator. Column chromatography in EtOAc/PE 3:1 provided 11 (138 mg, 57%) as a white amorphous solid. *R_{f}* 0.65 (EtOAc). ¹H NMR (CDCl₃, 400 MHz, ¹H-¹H COSY): *δ* 8.39 (ddd, 2H, *J* = 4.9, 1.8, 1.0 Hz, C*H*_{Py}), 8.17-8.11 (m, 4H, C*H*_{Bz}), 8.15 (s, 2H, C*H*_{Tria}), 7.94-7.86 (m, 8H, C*H*_{Bz}, 2H, C*H*_{Py}), 7.73-7.68 (m, 2H, C*H*_{Bz}), 7.64-7.54 (m, 6H, C*H*_{Bz}, 2H, C*H*_{Py}), 7.49-7.40 (m, 2H, C*H*_{Bz}), 7.40-7.30 (m, 8H, C*H*_{Bz}), 7.09 (ddd, 2H, *J* = 7.6, 4.9, 1.2 Hz, C*H*_{Py}), 6.01 (dd, 2H, *J=* 10.9, 9.8 Hz, H-2), 5.74 (dd, 2H, *J* = 3.2, 0.9 Hz, H-4), 5.17 (d, 2H, *J* = 9.8 Hz, H-1), 5.12 (dd, 2H, *J* = 10.9, 3.2 Hz, H-3), 4.62 (dd, 2H, *J* = 11.6, 7.7 Hz, H-6), 4.30 (dd, 2H, *J* = 11.6, 5.2 Hz, H-6'), 4.00 (ddd, 2H, *J =* 7.7, 5.2, 0.9 Hz, H-5). ¹³C{¹H} NMR (CDCl₃, 101 MHz, ¹H-¹³C HSQC, ¹H-¹³C HMBC): *δ* 165.8, 165.1, 165.0 (3×2*C*O), 149.6 (2×*C*H_{Py}), 149.4 (2×*C*_{q(Py)}), 148.6 (2×*C*_{q(Tria)}), 136.7 (2×*C*H_{Py}), 134.1, 134.0, 133.8 (3×2*C*H_{Bz}), 130.3 (2×*C*_{q(Bz)}), 130.21, 130.20, 130.1 (3×4*C*H_{Bz}), 129.7 (2×*C*_{q(Bz)}), 129.1 (2×2*C*H_{Bz}), 128.9 (2×*C*_{q(Bz)}), 128.6, 128.5 (2×4*C*H_{Bz}), 122.9 (2×*C*H_{Py}), 121.4 (2×*C*H_{Tria}), 120.2 (2×*C*H_{Py}), 82.2 (2×C-1), 76.3 (2×C-5), 69.5 (2×C-4), 67.3 (2×C-2), 63.6 (2×C-3), 62.2 (2×C-6). HRMS-ESI (m/z): [M + H]⁺ calculated for C₆₈H₅₅N₈O₁₄S, 1239.3558; found 1239.3555.

### Benzoylated ruthenium complex with p-cymene and Cl ligands and PF6 counterions (12)

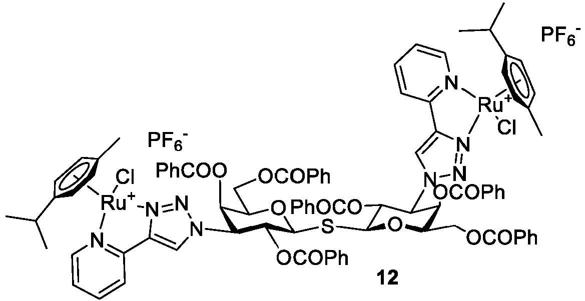

A dark red solution of dichloro(p-cymene)ruthenium dimer (68 mg, 0.11 mmol) in dichloromethane (3 mL) was added to a suspension of **11** (138 mg, 0.11 mmol) and KPF₆ (41 mg, 0.22 mmol) in a mixture of methanol/dichloromethane 1:1 (6 mL). A change in color from red to yellow occurred, the starting material completely dissolved and KCl precipitated. The mixture was stirred at room temperature for 10 minutes and filtered. The solvents were evaporated using a rotary vacuum evaporator, the crude mixture was dissolved in a minimal amount of dichloromethane, and the product **12** precipitated by a slow addition of methyl tert-butyl ether as a yellow solid (198 mg, 86%) mixture of 4 diastereomers. To obtain an analytical sample free from traces of residual solvents, the product was dissolved in a small amount of dichloromethane and distilled three times with chloroform. *R_{f}* 0.60 (CH₂Cl₂/CH₃OH 10:1). ¹H NMR (dmso-*d*₆, 400 MHz, ¹H-¹H COSY): *δ* 9.47, 9.46 (2×s, 4×2H, C*H*_{Tria}), 9.37-9.34 (m, 4×2H, C*H*_{Py}), 8.24-8.15 (m, 4×4H, C*H*_{Py}), 8.01-7.34 (m, 4×32H, C*H*_{Bz}, C*H*_{Py}), 6.22-6.15 (m, 4×6H, H-2, H-3, H-4), 5.90-5.88 (m, 2×2H, C*H*_{*p*-cym}), 5.83-5.77 (m, 4×2H, H-1), 5.60-5.58 (m, 4×2H, C*H*_{*p*-cym}), 5.57-5.54, 5.50-5.48, 5.32-5.31, 5.27-5.25, 5.12-5.12 (5×m, 5×2×2H, C*H*_{*p*-cym}), 4.91-4.83 (m, 4×2H, H-5), 4.61-4.56 (m, 4×2H, H-6a), 4.36-4.26 (m, 4×2H, H-6b), 2.33-2.25, 2.19-1.84 (2×m, 2×2×2H, C*H*_{*i*-Pr}), 1.94 (2×s, 2×6H, *Me*_{*p*-cym}), 1.85 (2×s, 2×6H, *Me*_{*p*-cym}), 0.74 (d, 2×6H, *J* = 6.9 Hz, *Me*_{*i*-Pr}), 0.70 (2×d, 2×6H, *J* = 6.9 Hz, *Me*_{*i*-Pr}), 0.09, 0.50, 0.49 (3×d, 4×6H, *J* = 6.9 Hz, *Me*_{*i*-Pr}). ¹³C{¹H} NMR (dmso-*d*₆, 101 MHz, ¹H-¹³C HSQC, ¹H-¹³C HMBC, ¹H-¹³C HSQC TOCSY): *δ* 165.23, 165.20, 165.18, 165.16, 164.70, 164.59, 164.58, 164.4, 164.21, 164.20 (4×12*C*O), 155.7, 155.6 (4×2C*H*_{Py}), 147.23, 147.17 (4×2*C*_{q(Py)}), 145.8, 145.4 (4×2*C*_{q(Tria)}), 140.3, 140.2 (4×2C*H*_{Py}), 134.5, 134.4, 133.8, 133.7 (4×6*C*H_{Bz}), 129.83, 129.81, 129.7, 129.63, 129.6, 129.38, 129.36, 129.02, 129.00, 128.9, 128.8, 128.6, 128.5, 128.41, 128.37, 128.19, 128.17, 127.91, 127.88 126.9, 126.4, 126.3, 126.2 (4×2*C*H_{Py}, 4×6×4CH_{Bz}), 122.6 (4×2*C*H_{Py}), 103.8 (2×2*C*_{q}Me), 103.21, 103.19, 103.0 (4×2*C*_{q}CH_{*i*-Pr}), 102.0, 101.9 (2×2*C*_{q}Me), 86.4, 85.9, 85.8, 85.5, 85.1, 84.6, 83.0, 82.8, 82.62, 82.55, 82.4, 82.3, 81.1 (4×2C-1, (4×8*C*H_{*p*-cym}), 74.44, 74.41, 74.33, 74.31 (4×2C-5), 69.4, 69.3, 68.92, 68.90 (4×2C-4), 67.9, 67.2 (4×2C-2), 63.88, 63.87 (4×2C-3), 61.32, 61.27, 61.22, 61.16 (4×2C-6), 30.14, 30.08 (4×2*C*H_{*i*-Pr}), 22.0, 21.7, 21.1, 20.7 (4×2*Me*_{*i*-Pr}), 18.2, 17.9 (4×2*Me*_{*p*-cym}). ³¹P{¹H} NMR (dmso-*d*₆, 162 MHz): *δ -* 161.09 (hept, ¹J(P-F) = 711.3 Hz). ¹⁹F NMR (dmso-*d*₆, 376 MHz): *δ* -71.42 (d, ¹J(F-P) = 711.3 Hz). HRMS-APCI (m/z): [M - 2 PF₆]²⁺ calculated for C₈₈H₈₂Cl₂N₈O₁₄Ru₂S 890.1562; found 890.1561.

### 1,6-anhydro-2,4-di-O-acetyl-3-azido-3-deoxy-β-D-galactopyranose (13)

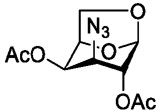

Compound **13** was prepared according to Mandal, S.; Nilsson, U. J., Tri-isopropylsilyl thioglycosides as masked glycosyl thiol nucleophiles for the synthesis of S-linked glycosides and glyco-conjugates. Organic & Biomolecular Chemistry 2014, 12 (27), 4816-4819.

### Phenyl 2,4,6-tri-O-acetyl-3-azido-1-thio-β-D-galactopyranoside (14)

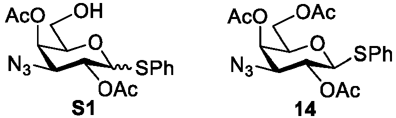

Trimethylsilyl thiophenol (1.4 mL, 7.14 mmol) and zinc iodide (1.27 g, 4.48 mmol) were sequentially added to a solution of 13 (600 mg, 2.24 mmol) in dichloroethane (20 mL), and the reaction mixture was stirred at room temperature overnight. The reaction mixture was diluted with dichloromethane (60 mL) and washed with water (80 mL). The aqueous phase was washed twice with 50 mL of dichloromethane. The organic phases were combined and dried over anhydrous sodium sulfate, and the crude mixture was concentrated on a rotary evaporator, dissolved in methanol (50 mL), acidified with 5 drops of acetic acid, and allowed to stir for 20 minutes. The solvents were evaporated on a rotary evaporator. Column chromatography in EtOAc/PE 2:3 first afforded phenyl 2,4-di-*O*-acetyl-3-azido-1-thio-α-D-galactopyranoside α-**S1** (130 mg, 15%), followed by phenyl 2,4-di-*O*-acetyl-3-azido-1-thio-β-D-galactopyranoside β-**S1** (630 mg, 74%). Ac₂O (30 mL, 0.27 mol) was added to the solution of β-**S1** (630 mg, 1.65 mmol) in pyridine (40 mL), and the mixture was left stirring overnight. Evaporation of the solvents and co-distillation with toluene (3×) provided **14** (700 mg, 100% yield for the acetylation step, 74% overall yield for both steps) as a white crystalline substance, mp 105-106 °C (EtOAc/PE), *R_{f}* 0.50 (EtOAc/PE 1:1). NMR spectra were in accordance with WO 2013/110704 A1.

### Methyl 4-O-(2,4,6-tri-O-acetyl-3-azido-3-deoxy-β-D-galactopyranosyl)-3,6-di-O-benzyl-2-deoxy-2-phtalimido-β-D-glucopyranoside (15)

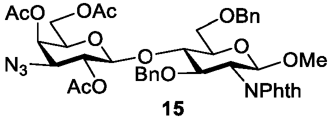

Methyl 3,6-di-*O*-benzyl-2-deoxy-2-phtalimido-β-D-glucopyranoside (prepared according to Kurfiřt, M.; Dračínský, M.; Červenková Št'astná, L.; Cuřínová, P.; Hamala, V.; Hovorková, M.; Bojarová, P.; Karban, J., Selectively Deoxyfluorinated N-Acetyllactosamine Analogues as 19F NMR Probes to Study Carbohydrate-Galectin Interactions. Chemistry A European Journal 2021, 27 (51), 13040-13051) (150 mg, 0.30 mmol) along with the glycosyl donor **14** (170 mg, 0.40 mmol) was co-distilled with toluene (3×) and dried under vacuum using an oil pump for 15 minutes. Anhydrous dichloromethane (10 mL) and 3 Å molecular sieves (1 g) were added under an argon atmosphere, and the mixture was stirred at room temperature for 1 hour. To the reaction mixture cooled to 0 °C, N-iodosuccinimide (134 mg, 0.60 mmol) and trifluoromethanesulfonic acid (20 µL, added dropwise) were added, and the mixture was stirred at 0 °C for 1 hour. The reaction mixture was diluted with dichloromethane (30 mL), a saturated solution of NaHCO₃ (till neutral pH) and a 10% solution of Na₂S₂O₃ (5 mL), filtered, transferred to a separatory funnel, and washed with water (30 mL). The aqueous phase was extracted twice with dichloromethane (30 mL). The combined organic phases were dried with anhydrous sodium sulfate, and the solvents were evaporated on a rotary vacuum evaporator. Column chromatography in EtOAc/PE 2:3 provided 15 (220 mg, 80%) as a colorless gel. *R_{f}* 0.50 (EtOAc/PE 1:1). NMR spectra are in accordance with Helland, A.-C.; Hindsgaul, O.; Palcic, M. M.; Stults, C. L.; Macher, B. A., Methyl 3-amino-3-deoxy-β-D-galactopyranosyl-(1→ 4)-2-acetamido-2-deoxy-β-D-glucopyranoside: An inhibitor of UDP-D-galactose: β-D-galactopyranosyl-(1→4)-2-acetamido-2-deoxy-D-glucose (1→3)-α-D galactopyranosyltransferase. Carbohydrate research 1995, 276 (1), 91-98.

### Methyl 4-O-(3-azido-3-deoxy-β-D-galactopyranosyl)-2-acetamido-2-deoxy-β-D-glucopyranoside (16)

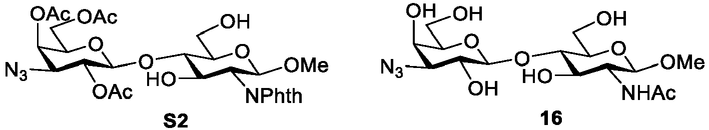

A solution of Na₂S₂O₄ (226 mg, 1.29 mmol) in water (8 mL) was slowly added dropwise to a mixture of **15** (210 mg, 0.26 mmol) and NaBrO₃ (196 mg, 1.29 mmol) in EtOAc (5 mL) and water (4 mL). The mixture was stirred vigorously for 6 hours. A changed of color from colorless to orange-red and back to colorless occured, indicating the end of the reaction. The mixture was diluted with dichloromethane (40 mL), and washed with water (30 mL). The aqueous phase was extracted four times with dichloromethane (30 mL). The combined organic phases were dried over anhydrous sodium sulfate, and the solvents were evaporated on a rotary vacuum evaporator. Column chromatography in EtOAc/PE 2:1 provided the intermediate product **S2** (145 mg, 89%) as a colorless gel. *R_{f}* 0.10 (EtOAc/PE 1:1). ¹H NMR (CDCl₃, 400 MHz, ¹H-¹H COSY): *δ* 7.84, 7.72 (2×dd, 2×2H, *J* = 5.3, 3.1 Hz, C*H*_{Ph}), 5.37 (d, 1H, *J* = 3.4 Hz, H-4'), 5.21 (dd, 1H, *J* = 10.6, 8.0 Hz, H-2'), 5.19 (d, 1H, *J* = 8.6 Hz, H-1), 4.64 (d, 1H, *J* = 8.0 Hz, H-1'), 4.42 (dd, 1H, *J=* 10.8, 8.4 Hz, H-3), 4.15-4.11 (m, 1H, H-6'a), 4.09 (dd, 1H, *J* = 10.8, 8.6 Hz, H-2), 3.99-3.94 (m, 2H, H-5', H-6'b), 3.91 (dd, 1H, *J* = 12.2, 2.1 Hz, H-6a), 3.72 (dd, 1H, *J* = 9.5, 8.4 Hz, H-4), 3.69 (dd, 1H, *J* = 12.2, 3.1 Hz, H-6b), 3.61 (dd, 1H, *J =* 10.6, 3.4 Hz, H-3'), 3.56 (ddd, 1H, *J* = 9.5, 3.1, 2.1 Hz, H-5), 3.44 (s, 3H, OMe), 2.20, 2.15, 1.85 (3×s, 3×3H, *M*e_{Ac}). ¹³C{¹H} NMR (CDCl₃, 101 MHz, ¹H-¹³C HSQC, ¹H-¹³C HMBC, ¹H-¹³C HSQC TOCSY): *δ* 170.6, 170.0, 169.5 (3×*C*O_{Ac}), from HMBC 168.1 (2*C*O_{Phth}), 134.2 (2*C*H_{Ph}), 131.9 (2*C*_{q}), 123.5 (2*C*H_{Ph}), 102.2 (C-1'), 99.5 (C-1), 82.2 (C-4), 74.2 (C-5), 72.5 (C-5'), 69.8 (C-3/2'), 67.7 (C-4'), 61.9 (C-6'), 61.8 (C-3'), 60.9 (C-6), 57.3 *(OMe),* 56.0 (C-2), 20.9, 20.7, 20.3 (3×*Me*_{Ac}). HRMS-APCI (m/z): [M - N₂ + H]⁺ calcd for C₂₇H₃₃N₂O₁₄, 609.1932; found 609.1929.

Ethylendiamine (1.5 mL, 22.46 mmol) was added to a solution of **S2** (300 mg, 0.47 mmol) in methanol (5 mL), and the mixture was refluxed for 4 hours. The solvents were evaporated, and the crude mixture was co-distilled with toluene (3×), dissolved in methanol (20 mL), Ac₂O (5 mL, 52.94 mmol) was added, and the reaction mixture was stirred for 2 hours. The solvents were evaporated on a rotary vacuum evaporator. Column chromatography in DCM/MeOH 6:1 provided 16 (178 mg, 89% for these 2 steps, 79% overall for all 3 steps) as white crystalline solid, mp 254-256 °C (decomp., EtOAc/MeOH). *R_{f}* 0.20 (DCM/MeOH 6:1). ¹H NMR (MeOH-*d*₄, 400 MHz, ¹H-¹H COSY): *δ* 4.45 (d, 1H, *J =* 7.7 Hz, H-1'), 4.32 (d, 1H, *J =* 8.3 Hz, H-1), 3.93 (dd, 1H, *J* = 12.2, 2.5 Hz, H-6a), 3.90 (dd, 1H, *J =* 3.1, 0.9 Hz, H-4'), 3.87 (dd, 1H, *J* = 12.2, 4.3 Hz, H-6b), 3.73 (dd, 1H, *J* = 11.5, 7.4 Hz, H-6'a), 3.75-3.61 (m, 6H, H-2, H-3, H-4, H-2', H-5', H-6'b), 3.46 (s, 3H, *OMe),* 3.40 (ddd, 1H, *J =* 9.2, 4.3, 2.5 Hz, H-5), 3.33 (dd, 1H, *J =* 10.6, 3.1 Hz, H-3'), 1.97 (s, 3H, *M*e_{Ac}). ¹³C{¹H} NMR (MeOH-*d*₄, 101 MHz, ¹H-¹³C HSQC, ¹H-¹³C HMBC): *δ* 173.6 *(CO), 105.1* (C-1'), 103.6 (C-1), 80.7 (C-4), 77.8 (C-3), 76.6 (C-5), 74.3 (C-5'), 70.7 (C-2'), 69.4 (C-4'), 66.7 (C-3'), 62.4 (C-6'), 61.8 (C-6), 57.0 (O*Me*)*,* 56.6 (C-2), 22.9 (*Me*_{Ac}). HRMS-APCI (m/z): [M -N₂ + H]⁺ calcd for C₁₅H₂₇N₂O₁₀, 395.1660; found 395.1956.

### Methyl 4-O-{3-[4-(pyridin-2-yl)-1H-1,2,3-triazol-1-yl]-3-deoxy-β-D-galactopyranosyl}-2-acetamido-2-deoxy-p-D-glucopyranoside (17)

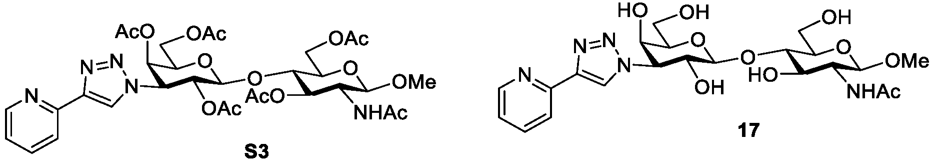

Ac₂O (5 mL, 52.94 mmol) was added to a solution of **16** (135 mg, 0.32 mmol) in pyridine (7 mL), and the mixture was stirred at room temperature overnight. The solvents were evaporated on a rotary vacuum evaporator, and the crude mixture was co-distilled with toluene (3×) and then dissolved again in dimethylformamide (2 mL). Copper(II) sulfate pentahydrate (16 mg, 0.06 mmol), ascorbic acid (11 mg, 0.06 mmol), and 2-ethynylpyridine (39 µL, 0.38 mmol) were added, and the reaction mixture was stirred at 40°C for 2 hours. After cooling the reaction mixture to room temperature, it was diluted with dichloromethane (30 mL) and washed with water (30 mL); the aqueous phase was extracted twice with dichloromethane (20 mL). The combined organic phases were dried over anhydrous sodium sulfate. The solvents were evaporated on a rotary vacuum evaporator. Column chromatography in EtOAc provided **S3** (209 mg, 89%) as a white amorphous solid.

A 0.1 M solution of sodium methanolate in methanol was added dropwise to a solution of S3 (209 mg, 0.28 mmol) in methanol (10 mL) until reaching pH = 9. The reaction mixture was stirred at room temperature overnight. Neutralization with acidic ion exchange resin DOVEX50W, filtration, and solvent evaporation yielded **17** (149 mg, 100% for this step, 89% overall yield for both steps) as a yellowish amorphous solid. *R_{f}* 0.35 (DCM/MeOH 3:1). ¹H NMR (MeOH-*d*₄, 400 MHz, ¹H-¹H COSY): *δ* 8.56 (br s, 2H, C*H*_{Py}, C*H*_{Tria}), 8.10 (d, 1H, *J* = 6.0 Hz, C*H*_{Py}), 7.93 (dd, 1H, *J* = 6.0, 8.4 Hz, C*H*_{Py}), 7.38 (br s, 1H, C*H*_{Py}), 4.90 (dd, 1H, *J =* 11.1, 3.2 Hz, H-3'), 4.68 (d, 1H, *J =* 7.5 Hz, H-1'), 4.35 (d, 1H, *J* = 8.2 Hz, H-1), 4.22 (dd, 1H, *J =* 11.1, 7.5 Hz, H-2'), 4.09 (d, 1H, *J =* 3.1 Hz, H-4'), 3.95 (dd, 1H, *J* = 12.2, 2.5 Hz, H-6a), 3.91-3.87 (m, 2H, H-6b, H-5'), 3.80 (dd, 1H, *J =* 11.4, 7.1 Hz, H-6'a), 3.76 (dd, 1H, *J* = 10.4, 8.2 Hz, H-2), 3.75-3.64 (m, 3H, H-3, H-4, H-6'b), 3.47 (s, 3H, *OMe),* 3.43 (ddd, 1H, *J =* 9.4, 4.2, 2.5 Hz, H-5), 1.98 (s, 3H, *Me*_{Ac}). ¹³C{¹H} NMR (MeOH-*d*₄, 101 MHz, ¹H-¹³C HSQC, ¹H-¹³C HMBC): *δ* 173.6 (CO), from HMBC 151.3 (*C*_{q(Py)}), 150.4 (*C*H_{Py}), from HMBC 148.1 (*C*_{q(Tria)}), 139.0 (*C*H_{Py}), from HSQC 124.4 (*C*H_{Py}), 123.8 (*C*H_{Tria}), from HSQC 121.7 (*C*H_{Py}), 105.3 (C-1'), 103.6 (C-1), 80.7 (C-4), 77.9 (C-5'), 76.6 (C-5), 74.4 (C-3), 69.5/69.4 (C-2'/4'), 67.7 (C-3'), 62.3 (C-6'), 61.7 (C-6), 57.1 (O*Me*)*,* 56.6 (C-2), 22.9 (*Me*_{Ac}). HRMS-ESI (m/z): [M + Na]⁺ calculated for C₂₂H₃₁N₅O₁₀Na, 548.1963; found 548.1971.

### LacNAc ruthenium complex with p-cymene and Cl ligands and Cl⁻ counterion (18)

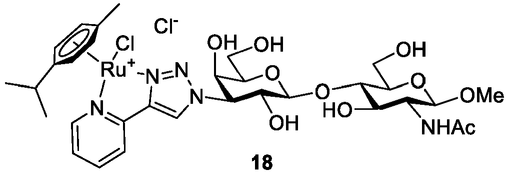

A dark red solution of dichloro(p-cymene)ruthenium dimer (18 mg, 0.03 mmol) in dichloromethane (1 mL) was added to a solution of 10 (31 mg, 0.06 mmol) in a mixture of methanol/dichloromethane 1:1 (2 mL). Almost immediately, a color change from red to yellow occurred. The mixture was stirred at room temperature for 10 minutes. The solvents were evaporated on a rotary vacuum evaporator, and the crude mixture was dissolved in a minimal amount of methanol. The product **18** precipitated by slow addition of methyl tert-butyl ether as a yellow solid (42 mg, 86%) mixture of two diastereomers in a ratio of 1:0.6. To obtain an analytical sample free from traces of residual solvents, the product was dissolved in a small amount of methanol and distilled three times with chloroform. *R_{f}* 0.25 (DCM/MeOH 3:1). Major diastereomer: ¹H NMR (MeOH-*d*₄, 400 MHz, ¹H-¹H COSY): *δ* 9.42-9.40 (m, 1H, C*H*_{Py}), 9.13 (s, 1H, C*H*_{Tria}), 8.20-8.14 (m, 2H, C*H*_{Py}), 7.68-7.64 (m, 1H, C*H*_{Py}), 6.12 (dd, 1H, *J* = 6.3, 1.2 Hz, C*H*_{*p*-cym}), 6.03, 5.92 (2×dd, 2×1H, *J* = 6.1, 1.2 Hz, C*H*_{*p*-cym}), 5.81 (dd, 1H, *J* = 6.3, 1.2 Hz, C*H*_{*p*-cym}), 5.16 (dd, 1H, *J* = 11.1, 3.1 Hz, H-3'), 4.74 (d, 1H, *J =* 7.6 Hz, H-1'), 4.38 (d, 1H, *J =* 8.2 Hz, H-1), 4.27 (dd, 1H, *J =* 11.1, 7.6 Hz, H-2'), 4.15 (dd, 1H, *J* = 3.1, 1.1 Hz, H-4'), 3.97-3.88 (m, 3H, H-6a, H-6b, H-5'), 3.85-3.65 (m, 5H, H-2, H-3, H-4, H-6'a, H-H-6'b), 3.47 (s, 3H, *OMe),* 3.45 (dt, 1H, *J* = 9.6, 3.3 Hz, H-5), 2.78-2.71 (m, 1H, C*H*_{*i*-Pr}), 2.23 (s, 3H, *Me*_{*p-*cym}), 1.99 (s, 3H, *Me*_{Ac}), 1.16, 1.06 (2×d, 2×3H, *J* = 6.9 Hz, *Me*_{*i*-Pr}). ¹³C{¹H} NMR (MeOH-*d*₄, 101 MHz, ¹H-¹³C HSQC, ¹H-¹³C HMBC, ¹H-¹³C HSQC TOCSY): *δ* 172.3 (*C*O)*,* 155.3 (C*H*_{py}), 148.6 (*C*_{q(Py)}), 146.3 (*C*_{q(Tria)}), 140.0, 125.9 (2×*C*H_{Py}), 124.5 (*C*H_{Tria}), 122.0 (*C*H_{Py}), 105.0 (*C*_{q}CH_{*i-*Pr}), 103.7 (C-1'), 102.5 (*C*_{q}Me), 102.2 (C-1), 85.9, 84.5, 84.1, 83.3 (4×*C*H_{*p*-cym}), 79.3 (C-4), 76.4 (C-5'), 75.2 (C-5), 72.9 (C-3), 68.2 (C-3'), 67.9 (C-2'), 67.7 (C-4'), 60.7 (C-6'), 60.4 (C-6), 55.7 *(OMe),* 55.5 (C-2), 30.8 (*C*H_{*i*-Pr}), 21.5 (*Me*_{Ac}), *21.2,* 20.4 (2×*Me*_{*i*-Pr}), 17.3 (*Me*_{*p*-cym}).

Minor diastereomer: ¹H NMR (MeOH-*d*₄, 400 MHz, H-H COSY): *δ* 9.42-9.40 (m, 1H, C*H*_{Py}), 9.09 (s, 1H, C*H*_{Tria}), 8.20-8.14 (m, 2H, C*H*_{Py}), 7.68-7.64 (m, 1H, C*H*_{Py}), 6.11 (dd, 1H, *J* = 6.3, 1.2 Hz, C*H*_{*p*-cym}), 6.06, 5.89 (2×dd, 2×1H, *J* = 6.1, 1.2 Hz, C*H*_{*p*-cym}), 5.83 (dd, 1H, *J* = 6.3, 1.2 Hz, C*H*_{*p*-cym}), 5.09 (dd, 1H, *J* = 11.0, 2.9 Hz, H-3'), 4.73 (d, 1H, *J=* 7.5 Hz, H-1'), 4.37 (d, 1H, *J* = 8.2 Hz, H-1), 4.34 (dd, 1H, *J=* 11.0, 7.5 Hz, H-2'), 4.20 (dd, 1H, *J* = 2.9, 1.1 Hz, H-4'), 3.97-3.88 (m, 3H, H-6a, H-6b, H-5'), 3.85-3.65 (m, 5H, H-2, H-3, H-4, H-6'a, H-6'b), 3.47 (s, 3H, *OMe),* 3.44 (dt, 1H, *J* = 9.6, 3.3 Hz, H-5), 2.78-2.71 (m, 1H, C*H*_{*i*-Pr}), 2.22 (s, 3H, *Me*_{*p*-cym}), 1.15, 1.09 (2×d, 2×3H, *J* = 6.9 Hz, *Me*_{*i*-Pr}). ¹³C{¹H} NMR (MeOH-*d*₄, 101 MHz, HSQC, HMBC, HSQCTOCSY): *δ* 172.3 (*C*O), 155.3 (C*H*_{Py}), 148.6 (*C*_{q(Py)}), 146.2 (*C*_{q(Tria)}), 140.0, 125.9 (2×*C*H_{Py}), 124.8 (*C*H_{Tria}), 122.0 (*C*H_{Py}), 105.0 (*C*_{q}CH_{*i*-Pr}), 103.7 (C-1'), 102.5 (*C*_{q}Me), 102.2 (C-1), 86.0, 84.6, 84.0, 83.2 (4×*C*H_{*p*-cym}), 79.2 (C-4), 76.2 (C-5'), 75.2 (C-5), 73.0 (C-3), 68.5 (C-3'), 68.0 (C-4'), 67.5 (C-2'), 60.7 (C-6'), 60.3 (C-6), 55.7 *(OMe),* 55.4 (C-2), 30.9 (*C*H_{*i-*Pr}), 21.5 (*Me*_{Ac}), 21.2, 20.5 (2×*Me*_{*i*-Pr}), 17.4 (*Me*_{*p-*cym}). HRMS-APCI (m/z): [M - Cl]⁺ calculated for C₃₂H₄₅ClN₅O₁₀Ru, 796.1899; found 796.1904.

### LacNAc ruthenium complex with p-cymene and Cl ligands and PF₆⁻ counterion (19)

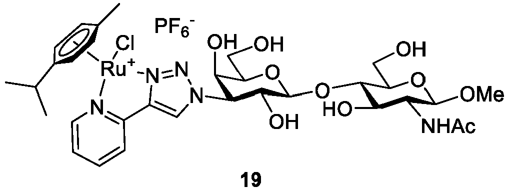

A dark red solution of dichloro(p-cymene)ruthenium dimer (83 mg, 0.14 mmol) in dichloromethane (4 mL) was added to a suspension of **17** (142 mg, 0.27 mmol) and KPF₆ (50 mg, 0.27 mmol) in a mixture of methanol/dichloromethane 1:1 (8 mL). Almost immediately, a color change from red to yellow occurred. The mixture was stirred at room temperature for 10 minutes. The solvents were evaporated on a rotary vacuum evaporator, and the crude mixture was dissolved in a minimal amount of methanol. The product **19** precipitated by slow addition of methyl *tert-*butyl ether as a yellow solid (186 mg, 73%) mixture of two diastereomers in a ratio of 1:0.6. To obtain an analytical sample free from traces of residual solvents, the product was dissolved in a small amount of methanol and distilled three times with chloroform. *R*_{f} 0.35 (DCM/MeOH 3:1). Major diastereomer: ¹H NMR (dmso-*d*₆, 400 MHz, ¹H-¹H COSY): *δ* 9.47-9.46 (m, 1H, C*H*_{Py}), 9.39 (s, 1H, C*H*_{Tria}), 8.30-8.22 (m, 2H, C*H*_{Py}), 7.84 (d, 1H, *J* = 7.5 Hz, NH), 7.70-7.66 (m, 1H, C*H*_{Py}), 6.14 (dd, 1H, *J* = 6.0, 1.2 Hz, C*H*_{*p*-cym}), 6.07, 6.02 (2×dd, 2×1H, *J* = 6.2, 1.2 Hz, C*H*_{*p*-cym}), 5.86 (dd, 1H, *J* = 6.0*,* 1.2 Hz, C*H*_{*p*-cym}), 5.86 (d, 1H, *J* = 6.2 Hz, O*H*-2'), 5.73 (d, 1H, *J* = 6.5 Hz, O*H*-4'), 5.18 (dd, 1H, *J =* 11.0, 3.1 Hz, H-3'), 4.89 (t, 1H, *J* = 5.0 Hz, O*H*-6'), 4.76 (t, 1H, *J* = 6.2 Hz, OH-6), 4.67 (d, 1H, *J =* 7.5 Hz, H-1'), 4.61 (d, 1H, *J* = 2.1 Hz, OH-3), 4.28 (d, 1H, *J =* 7.6 Hz, H-1), 4.14-4.04 (m, 1H, H-2'), 3.98 (dd, 1H, *J =* 6.5, 3.1 Hz, H-4'), 3.85 (t, 1H, *J =* 5.6 Hz, H-5'), 3.80-3.76 (m, 1H, H-6a), 3.71-3.65 (m, 1H, H-6b), 3.60-3.46 (m, 5H, H-2, H-3, H-4, H-6'a, H-6'b), 3.35 (s, 3H, *OMe),* 3.33-3.29 (m, 1H, H-5), 2.70-2.61 (m, 1H, C*H*_{*i-*Pr}), 2.14 (s, 3H, *Me*_{*p*-cym}), 1.81 (s, 3H, *Me*_{Ac}), 1.07, 0.93 (2×d, 2×3H, *J* = 6.9 Hz, *Me*_{*i*-Pr})*.* ¹³C{¹H} NMR (dmso-*d*₆, 101 MHz, ¹H-¹³C HSQC, ¹H-¹³C HMBC, ¹H-¹³C HSQCTOCSY): *δ* 169.9 (CO), 155.7 (C*H*_{py}), 148.1 (*C*_{q(Py)}), 145.6 (*C*_{q(Tria)}), 140.2, 125.9 (2×*C*H_{Py}), 125.4 (*C*H_{Tria}), 122.0 (*C*H_{Py}), 104.1 (*C*_{q}CH_{*i*-Pr}), 103.5 (C-1'), 102.0 (C-1), 101.7 (*C*_{q}Me), 85.5, 84.5, 83.8, 82.9 (4×*C*H_{*p*-cym}), 80.7 (C-4), 76.0 (C-5'), 75.0 (C-5), 72.4 (C-3), 67.61/67.56 (C-2'/3'), 66.8 (C-4'), 60.2 (C-6), 60.0 (C-6'), 55.8 *(OMe),* 54.7 (C-2), 30.4 (*C*H_{*i*-Pr}), 23.1 (*Me*_{Ac}), 22.0, 21.2 (2×*Me*_{*i*-Pr}), 18.1 (*Me*_{*p*-cym}). ¹⁹F NMR (dmso-*d*₆, 376 MHz): *δ* -71.43 (d, ¹*J*_{(F-P)} = 711.4 Hz). ³¹P NMR (dmso-*d*₆, 162 MHz): *δ* -161.98 (hept, ¹*J*_{(P-F)} = 711.4 Hz). Minor diastereomer: ¹H NMR (dmso-*d*₆, 400 MHz, ¹H-¹H COSY): *δ* 9.47-9.46 (m, 1H, C*H*_{Py}), 9.37 (s, 1H, C*H*_{Tria}), 8.30-8.22 (m, 2H, C*H*_{Py}), 7.84 (d, 1H, *J =* 7.5 Hz, NH), 6.17, 6.13, 5.98, 5.92 (4×d, 4×1H, *J =* 6.2 Hz, C*H*_{*p*-cym}), 5.86 (d, 1H, *J* = 6.2 Hz, OH-2'), 5.65 (d, 1H, *J =* 6.4 Hz, O*H-*4'), 5.15 (dd, 1H, *J =* 11.2, 3.0 Hz, H-3'), 4.96 (t, 1H, *J =* 4.9 Hz, O*H*-6'), 4.75 (dd, 1H, *J =* 6.2, 4.1 Hz, OH-6), 4.67 (d, 1H, *J =* 7.5 Hz, H-1'), 4.60 (d, 1H, *J =* 2.0 Hz, OH-3), 4.27 (d, 1H, *J =* 7.3 Hz, H-1), 4.14-4.04 (m, 2H, H-2', H-4'), 3.86 (dd, 1H, *J = 5.9,* 4.7 Hz, H-5'), 3.80-3.76 (m, 1H, H-6a), 3.71-3.65 (m, 1H, H-6b), 3.60-3.46 (m, 5H, H-2, H-3, H-4, H-6'a, H-6'b), 3.35 (s, 3H, *OMe),* 3.33-3.29 (m, 1H, H-5), 2.70-2.61 (m, 1H, C*H*_{*i*-Pr}), 2.12 (s, 3H, *Me*_{*p-*cym}), 1.81 (s, 3H, *Me*_{Ac}), 1.07, 1.00 (2×d, 2×3H, *J =* 6.9 Hz, *Me*_{*i-*Pr}). ¹³C{¹H} NMR (dmso-*d*₆, 101 MHz, ¹H-¹³C HSQC, ¹H-¹³C HMBC, ¹H-¹³C HSQC TOCSY): *δ* 168.9 (*C*O), 155.7 (C*H*Py), 148.2 (*C*_{q(Py)}), 145.3 (*C*_{q(Tria)}), 140.2, 125.9 (2×*C*H_{Py}), 125.4 (*C*H_{Tria}), 121.9 (*C*H_{Py}), 104.0 (*C*_{q}CH_{*i*-Pr}), 103.4 (C-1'), 101.9 (C-1), 101.7 (*C*_{q}Me), 85.7, 85.6, 83.6, 82.8 (4×CH_{*p*-cym}), 80.5 (C-4), 75.7 (C-5'), 75.0 (C-5), 72.4 (C-3), 67.61/67.56 (C-2'/3'), 67.0 (C-4'), 60.2 (C-6), 59.7 (C-6'), 55.8 (O*Me*)*,* 54.7 (C-2), 30.4 (*C*H_{*i*-Pr}), 23.1 (*Me*_{Ac}), 22.0, 21.3 (2×*Me*_{*i-*Pr}), 18.1 (*Me*_{*p-*cym}). ¹⁹F NMR (dmso-*d*₆, 376 MHz): *δ* -71.43 (d, ¹*J*_{(F-P)} = 711.4 Hz). ³¹P NMR (dmso-*d*₆, 162 MHz): *δ* -161.98 (hept, ¹*J*_{(P-F)} = 711.4 Hz). HRMS-APCI (m/z): [M - PF₆]⁺ calculated for C₃₂H₄₅ClN₅O₁₀Ru, 796,1899; found 796,1897.

Example 3: *Affinity determination of compounds **4-10** and **18-19** to human galectins 1 and 3.* The inhibitory potential of prepared compounds **4-10, 18-19,** and the standard **GB139** was determined using fluorescence anisotropy according to Sörme, P.; Kahl-Knutsson, B.; Huflejt, M.; Nilsson, U. J.; Leffler, H., Fluorescence polarization as an analytical tool to evaluate galectin-ligand interactions. Analytical Biochemistry 2004, 334 (1), 36-47, page 39, paragraph "Fluorescence Polarization". As a competitive fluorescent probe, the compound 3,3'-dideoxy-3-[4-(fluorescein-5-yl-carbonylamino-methyl)-1*H*-1,2,3-triazol-1-yl]-3'-[4-(3-fluorophenyl)-1H-1,2,3-triazol-1-yl]-1,1'-sulfanediyl-di-β-D-galactopyranoside was used in the measurements. Fluorescence anisotropy was measured using a Tecan Spark multimode microplate reader equipped with fluorescence polarization filters and black polystyrene round-bottomed microtiter plates (Corning^{®}, 384 wells) in samples with a final volume of 200 µL. The samples were incubated in the dark for 30 minutes before measurement. The dissociation constant (*K*_{d}) of the fluorescent probe was determined using a series of measurements with constant concentrations of the probe (50 nM for galectin 1 and 5 nM for galectin 3) and increasing concentrations of galectin (6 nM-60000 nM for galectin 1 and 0.3 nM-3000 nM for galectin 3).

The *K*_{d} values were obtained using nonlinear regression performed by GraphPad Prism software (version 10.0.2, GraphPad, USA) for direct ligand-protein interaction using the formula: A = A₀ + (Aₘₐₓ-A₀) × ([G]/(*K*_{d} + [G])), where A₀ is the anisotropy of the probe in the absence of galectin, Aₘₐₓ is the maximum achieved anisotropy of the probe with increasing galectin concentration, [G] is the total galectin concentration in a measured sample, and A is the measured anisotropy of the measured sample. Inhibition assays of prepared compounds to galectin 1 were performed with a constant protein concentration of 500 nM, a constant probe concentration of 100 nM, and increasing concentrations of the prepared inhibitor (1 nM-10000 nM for compounds **4** and **6,** 10 nM-4000 nM for compound **5,** 10 nM-10000 nM for compounds **7-10** and GB139, 10 nM-100000 nM for compound **18,** and 75 nM-30000 nM for compound **19).** Inhibition assays of prepared compounds to galectin 3 were performed with a constant protein concentration of 50 nM, a constant probe concentration of 5 nM, and increasing concentrations of the prepared inhibitor (0.1 µM-2500 µM for compounds **4, 6, 18** and **19,** 3.5 µM-400 µM for compound 5, 0.1 µM-500 µM for compounds **7-10,** and 1 nM-10000 nM for GB139). Raw anisotropy data from the reader were plotted against concentration using GraphPad Prism sowtfare (version 10.0.2, GraphPad, USA) and nonlinear fitting *"[Inhibitor] vs. response -- Variable slope (four parameters)"* analysis was used to calculate the *IC*₅₀ values (half-maximal inhibitory concentration) for each titration. The *IC*₅₀ values from each titration experiment were then used to calculate the inhibition constant *K*ᵢ using an online calculator (http://websites.umich.edu/~shaomengwanglab/software/calc_ki/index.html). The used model is described in the following publication: Development and optimization of a binding assay for the XIAP BIR3 domain using fluorescence polarization, Analytical Biochemistry 2004, 332 (2), 261-273. The measured data compared to the known galectin inhibitor **GB139** are displayed in Table 1. Compounds **5, 6,** and **7** exhibit significantly better affinity to galectin-1 than used fluorescent probe, and it is not possible to determine the exact value of *K*ᵢ with this method. It is therefore provided as a possible range of values.

**Table 1: Kᵢ of prepared compounds 4-10, 18-19, and the standard GB139 against human galectins 1 and 3 determined by fluorescence anisotropy. The listed values are averages and standard deviations from at least three independent measurements.**

| **Compound** | ***IC*₅₀ against human galectin-1 under measured condition (µM)** | ***K*ᵢ against human galectin-1 (µM)** | ***IC*₅₀ against human galectin-3-CRD under measured condition (µM)** | ***K*ᵢ against human galectin 3-CRD (µM)** |
|---|---|---|---|---|
| **4** | 0.328 ± 0.018 | 0.018 ± 0.011 | 100 ± 12 | 21.2 ± 2.7 |
| **5** | 0.187 ± 0.004 | <0.005 | 50.7 ± 9.0 | 10.7 ± 1.9 |
| **6** | 0.231 ± 0.017 | <0.005 | 71.7 ± 0.84 | 15.2 ± 0,2 |
| **7** | 0.307 ± 0.033 | <0.005 | 16.8 ± 1.5 | 3.54 ± 0.32 |
| **8** | 0.498 ± 0.026 | 0.114 ± 0.011 | 205 ± 63 | 43.4 ± 13 |
| **9** | 0.370 ± 0.016 | 0.037 ± 0.009 | 89.8 ± 16 | 19.0 ± 3.3 |
| **10** | 0.347 ± 0.013 | 0.023 ± 0.07 | 73.5 ± 8.1 | 15.6 ± 1.8 |
| **18** | 2.19 ± 0.24 | 1.17 ± 0,16 | 790 ± 20 | 167.2 ± 4.2 |
| **19** | 1.16 ±0.21 | 0.464 ± 0.11 | 152 ± 12 | 32.2 ± 2.5 |
| **GB139** | 0.423 ± 0.043 | 0.077 ± 0,027 | 0.079 ± 0.002 | 0.008 ± 0.001 |

The inhibitory potential of the prepared compounds **4, 6,** and standard GB139 against human galectins 1 and 3 was also determined using competitive enzyme-linked immunosorbent assay (ELISA). An immobilized competitive ligand, asialofetuin (ASF), a glycoprotein terminated with LacNAc residues interacting with galectins, was used. Wells of microtiter plates (96-well BRAND plates-F-pureGrade, BRAND, Germany) were coated with ASF (5 mg/ml in 0.1M CO₃²⁻/HCO₃⁻buffer pH 9-9.8; 50 µL per well) and incubated overnight in a humid chamber at 40 °C. Subsequently, wells were washed with 1 x 200 µL PBS (137 mM NaCl, 2.7 mM KCl, 8 mM Na₂HPO₄, and 2 mM KH₂PO₄, pH 7.4). PBS-Tween washes were performed 3 x 5 min following each subsequent step. The wells were blocked with 3% BSA in PBS (200 µL per well) and incubated for 1 hour at room temperature. Increasing concentrations of inhibitors in PBS buffer were mixed with His₆-GST-Gal-3 or His₆-GST-Gal-1 (target concentration 5 or 10 µM) and incubated in wells with ASF for 1 hour. Anti-GST antibody conjugated with horseradish peroxidase (prepared in-house) diluted 1:1000 in 1% BSA in PBS was used for labeling the N-terminal GST-tag of galectins and subsequent colorimetric immunodetection. 3,3',5,5'-tetramethylbenzidine (TMB) (0.1 mg/mL TMB in citrate phosphate buffer pH 6.0: 75 mM Na₂HPO₄, 25 mM citric acid; 50 µL per well) was used as the substrate for the peroxidase reaction and incubated until visible blue color developed (20 min). The reaction was stopped by addition of 1 M H₂SO₄ solution (50 µL per well), resulting in a color shift to yellow. The signal intensity was measured spectrophotometrically using the Multidetection reader Infinititte M1000 PRO (Tecan Group Ltd, CH) at 450 nm and corresponds to the amount of bound galectin. The respective inhibitory constant (*IC*₅₀) was calculated from nonlinear regression (dose-response inhibition slope) of sigmoidal curves using GraphPad Prism sowtfare (version 10.0.2, GraphPad, USA). The measured data compared to the known galectin inhibitor GB139 are shown in Table 2.

**Table 2: IC₅₀ values of compounds 4, 6, and standard GB139 against human galectins 1 and 3 determined by ELISA. The listed values are averages and standard deviations from at least 3 independent measurements.**

| **Compound:** | ***IC₅₀* against human galectin-1 (µM)** | ***IC₅₀* against human galectin-3 (µM)** |
|---|---|---|
| **GB139** | 0.148 ± 0.059 | 0.028 ± 0.016 |
| **4** | 0.026 ± 0.004 | 87.2 ± 4.4 |
| **6** | 0.006 0 ± 0.000 8 | 103.8 ± 38 |

### Example 4: Cytotoxicity of compounds 4, 6, 12, and 18.

The cytotoxic properties of the prepared compounds **4, 6, 12,** and **18** against tumor and non-tumor cell lines were determined using the MTT assay and expressed as *IC*₅₀ values. This assay is based on the reduction of the yellow soluble 3-[4,5-dimethylthiazol-2-yl]-2,5-diphenyl tetrazolium bromide (MTT) to insoluble formazan (blue star-shaped crystals). The reaction takes place on the mitochondrial membrane of living cells. Subsequently, the formazan is dissolved by adding a strong detergent (dimethyl sulfoxide, DMSO), and the color is evaluated spectrophotometrically at a wavelength of 570-595 nm. The absorbance value of the solution corresponds to the amount of live cells (the darker the color and hence higher absorbance, the higher the percentage of live and metabolically active cells).

Human cell lines derived from ovarian carcinoma (A2780, SK-OV-3), breast carcinoma (MDA-MB-231, MCF-7), and non-tumor epithelial line MCF-10A were used for the cytotoxicity test. Additionally, a cell line derived from embryonic kidney cells HEK293 was used as a control. A2780 cells were cultured in RPMI 1640 medium (Sigma-Aldrich) containing 10% fetal bovine serum (Biochrom AG), 2 mM L-glutamine (Gibco), and 100 U/mL penicillin/streptomycin (Biosera). SK-OV-3 cells were cultured in McCoy's medium (Sigma-Aldrich) containing 10% fetal bovine serum (Biochrom AG) and 100 U/mL penicillin/streptomycin (Biosera). MDA-MB-231, MCF-7, and HEK293 cell lines were cultured in HG-DMEM medium (Sigma-Aldrich) containing 10% fetal bovine serum (Biochrom AG), 100 U/mL penicillin/streptomycin (Biosera), and 100 mg/L pyruvate (Invitrogen). The MCF-10A cell line was cultured in D-MEM F12 medium (Gibco) containing 5% horse serum (Gibco), 20 ng/mL EGF (Sigma), 100 µg/mL hydrocortisone (UVAB Pharma), 10 µg/mL insulin (Eli Lilly), and 100 U/mL penicillin/streptomycin (Biosera).

The test proceeds according to the following procedure: Cells were seeded into the wells of a 96-well plate at a density of 5000 cells/100 µL (MDA-MB-231, MCF-7, SK-OV-3, HEK293), 8000 cells/100 µl (A2780, MCF-10A) in culture medium and incubated overnight in a CO₂ incubator at 37 °C, 5% CO₂. The next day, 50 µL of the test compound was added to the wells to a final concentration range of the compound: 100 µM, 25 µM, 10 µM, 5 µM, 2.5 µM, 1 µM, 0.2 µM, 0.05 µM), and the cells were incubated in the CO₂ incubator for 24-72 hours. Then 20 µL of MTT solution (2.5 mg/1 ml PBS) was added to the wells, and the cells were incubated for 3 hours in the CO₂ incubator at 37 °C, 5% CO₂. After incubation, the medium was removed, and 50 µL of DMSO was added. After dissolving the blue crystals, the absorbance was measured in the wells of the plate on an ELISA Reader (Multidetection reader Infinititte M1000 PRO (Tecan Group Ltd, CH)) at a wavelength of 570-595 nm. Evaluation of *IC₅₀* as the inflection point of the curve of measured absorbance values using the GraphPad Prism software (version 10.0.2, GraphPad, USA). The determined *IC*₅₀ values are displayed in Table 3.

**Table 3: IC₅₀ values of compounds 4, 6, 12, and 18 against selected tumor and non-tumor cell lines determined using the MTT assay. The listed values are averages and standard deviations from at least 3 independent measurements.**

| **Compound** | **HEK-293 (µM)** | **A2780 (µM)** | **SK-OV-3 (µM)** | **MDA-MB-231 (µM)** | **MCF-7 (µM)** | **MCF-10A (µM)** |
|---|---|---|---|---|---|---|
| **4** | 158 ± 42 | >200 | >200 | >200 | >200 | >200 |
| **6** | 174 ± 28 | n.d. | >200 | 141 ± 8 | n.d. | n.d. |
| **12** | 1.84 ± 0.20 | 2.34 ± 0.73 | 4.76 ± 1.17 | 11.40 ± 1.95 | 2.82 ± 0.57 | 2.32 ± 0.32 |
| **18** | >200 | >200 | >200 | >200 | n.d. | n.d. |

## Claims

1. A compound of general formula (I) 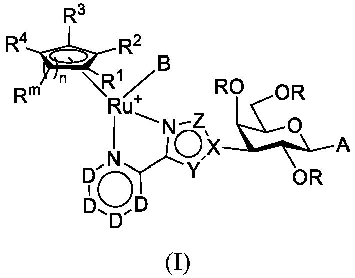 wherein
R¹, R², R³, R⁴, and R^{m} are independently selected from the group consisting of H and (C₁ to C₆) alkyl;
*n* is 1 or 2;
X and Z are N;
D and Y are CH;
R is independently H or an acyl of general formula (G) 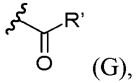
wherein R' is selected from the group comprising C₁-C₆ alkyl, C₃-C₈ cycloalkyl, and C₆-C₁₂ aryl;
A is selected from the group consisting of general formula (II) and (III) 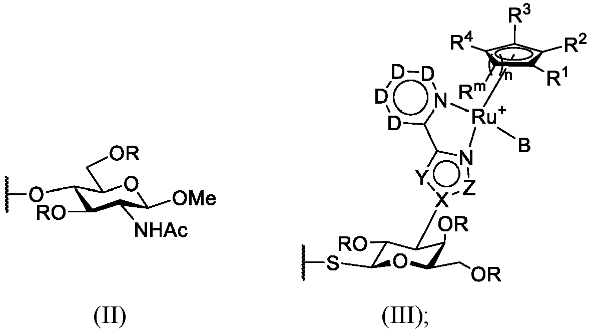
B is selected from the group comprising monovalent negatively charged ligands and neutral ligands, preferably B is selected from the group comprising F⁻, Cl⁻, Br⁻, I⁻, CN⁻, SNC⁻, OCN⁻, NO₂⁻, NO₃⁻, HSO₄⁻, OH⁻, CH₃COO⁻, CO, CO₂, CH₃CN, H₂O, NH₃, monovalent organic N-ligands, and organophopsphine ligands;
and wherein the positive charge of the compound of general formula (I) is compensated by an anion, preferably selected from the group comprising F⁻, Cl⁻, Br, I⁻, CN⁻, SCN⁻, OCN⁻, PF₆⁻, Zn₂Cl₆²⁻, BF₄⁻, NO₂⁻, NO₃⁻, HSO₄⁻, SO₄²⁻, H₂PO₄⁻, HPO₄²⁻, PO₄³⁻, OH⁻ and CH₃COO⁻.

2. The compound of general formula (I) according to claim 1, wherein R¹, R², R³, R⁴, and R^{m} are independently selected from the group consisting of H, -CH₃, and -CH(CH₃)₂.

3. The compound of general formula (I) according to any one of the preceding claims, wherein the structural unit (IV) 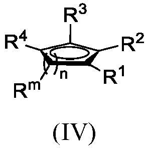 is selected from the group comprising cyclopentadiene, pentamethylcyclopentadiene, benzene, p-cymene, 1,3,5-trimethylbenzene, and hexamethylbenzene.

4. The compound of general formula (I) according to any one of the preceding claims, wherein said compound is selected from the group consisting of: 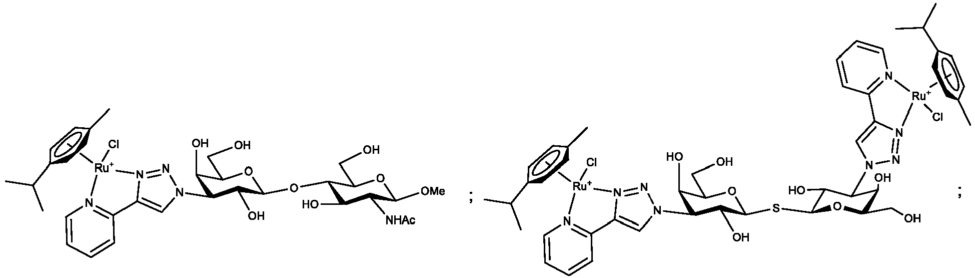 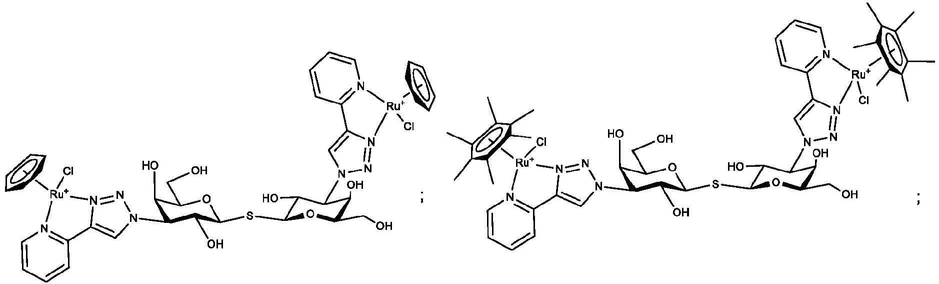 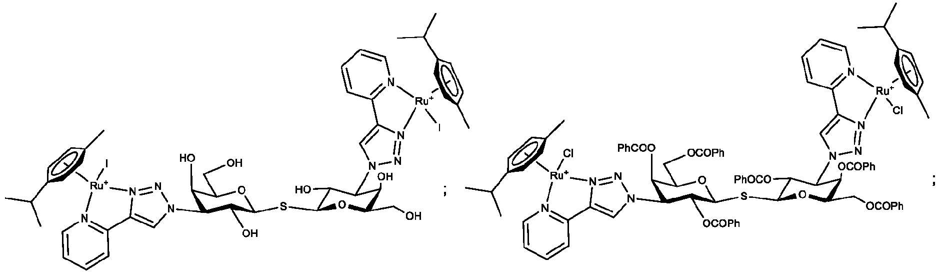 wherein the positive charge of the compound is compensated by an anion selected from the group comprising F⁻, Cl⁻, Br⁻, I⁻, CN⁻, SCN⁻, OCN⁻, PF₆⁻, Zn₂Cl₆²⁻, BF₄⁻, NO₂⁻, NO₃⁻, HSO₄⁻, SO₄²⁻, H₂PO₄⁻, HPO₄²⁻, PO₄³⁻, OH⁻ and CH₃COO⁻.

5. A method of preparing a compound of the general formula (Ia): 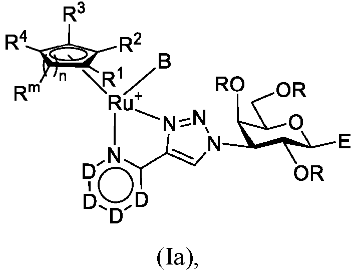
wherein B, D, R, R¹, R², R³, R⁴ a R^{m} are as defined in claim 1;
and wherein E is 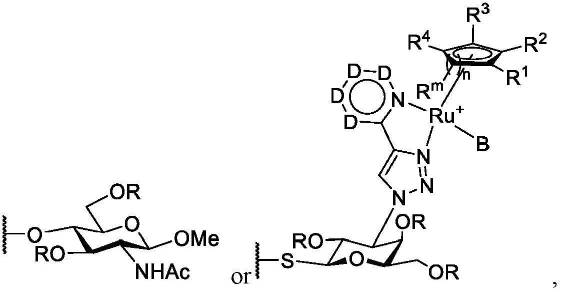
**characterized in that** it comprises the following steps:
(a) Huisgen cycloaddition of the azide precursor of the general formula (V) 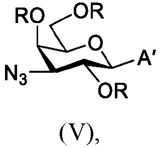
wherein R is as defined in claim 1, preferably R is H or CH₃C(=O)-; and
A' is 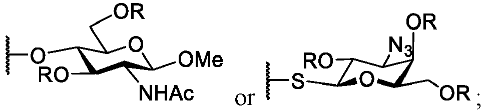
with the ethynyl derivative of a six-membered nitrogenous heterocycle of general formula (VII),
wherein D is defined in claim 1, 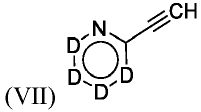
resulting in the formation of an intermediate of general formula (VIa) 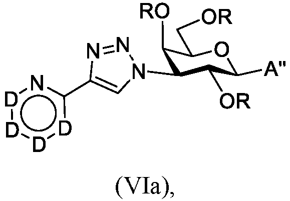
wherein R and D are defined in claim 1, and A" is 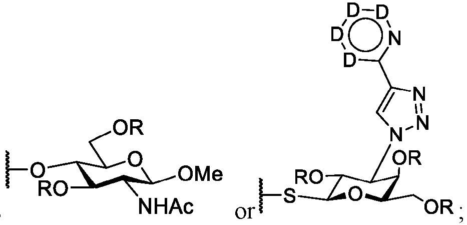
(b) if R is not H, optional step of hydrolysis of the OR groups to form an intermediate of general formula (VIb) 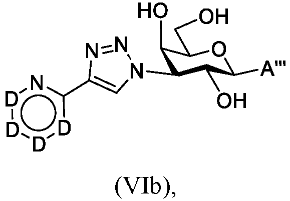
wherein D is as defined in claim 1, and
A‴ is 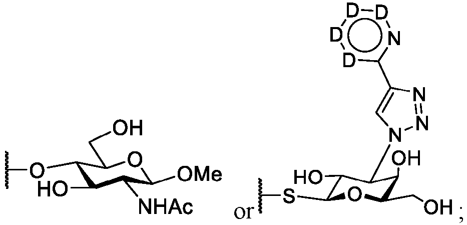
(c) a reaction of the compound of general formula (VIb) with a ruthenium complex of general formula [RuLB₂]₂, wherein L is the structural unit (IV) as defined in claim 3, and B is as defined in claim 1, to form the compound of general formula (Ia).

6. The method according to claim 5, wherein between the step (a) or, if present step (b), and the step (c), the compound of general formula (VIb) reacts with an acyl chloride of formula R'-C(=O)Cl to form an intermediate of general formula (VIc) 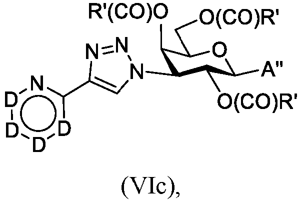
wherein R' and D are defined in claim 1, and
A" is 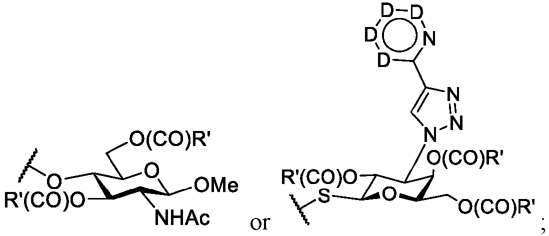
the step (c) is then performed with said compound of general formula (VIc) to give the compound of general formula (Ia).

7. Use of the compound of general formula (I) according to any of the preceding claims 1 to 4 as an analytical probe for *in vitro* determining the affinity of ligands to galectins, especially to galectin-1.

8. The compound of general formula (I) according to any of the preceding claims 1 to 4 for use as a medicament.

9. The compound of general formula (I) according to any of the preceding claims 1 to 4 for use in the treatment of diseases associated with galectin overexpression, especially galectin 1 overexpression, preferably selected from the group comprising cancer, fibrotic diseases, and HIV-1 infection.

10. The compound of general formula (I) according to any of the preceding claims 1 to 4 for use in the therapeutic inhibition of galectins *in vivo.*

11. Use of the compound of general formula (I) according to any of the preceding claims 1 to 4 for the inhibition of galectins *in vitro,* especially for the inhibition of galectin-1.

## Patentansprüche

1. Verbindung der allgemeinen Formel (I) 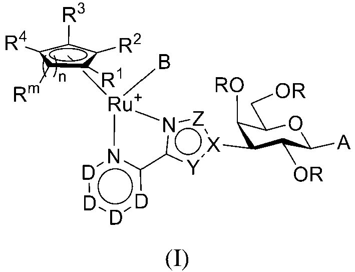 wobei
R¹, R², R³, R⁴ und R^{m} unabhängig voneinander aus der Gruppe bestehend aus H und (C₁-C₆)-Alkyl ausgewählt sind;
n ist 1 oder 2;
X und Z sind N;
D und Y sind CH;
R ist unabhängig H oder ein Acyl der allgemeinen Formel (G) 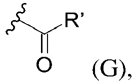
wobei R' ausgewählt ist aus der Gruppe bestehend aus C₁-C₆-Alkyl, C₃-C₈-Cycloalkyl und C₆-C₁₂-Aryl;
A ausgewählt ist aus der Gruppe bestehend aus den allgemeinen Formeln (II) und (III) 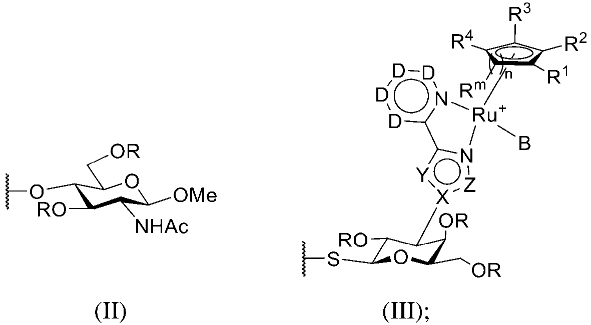
B ist ausgewählt aus der Gruppe der einwertigen, negativ geladenen Liganden und der neutralen Liganden, vorzugsweise ist B ausgewählt aus der Gruppe der Liganden F⁻, Cl⁻, Br⁻, I⁻, CN⁻, SNC⁻, OCN⁻, NO₂⁻, NO₃⁻, HSO₄⁻, OH⁻, CH₃COO⁻, CO, CO₂, CH₃CN, H₂O, NH₃, einwertigen organischen N-Liganden und Organophopsphin-Liganden;
wobei die positive Ladung der Verbindung der allgemeinen Formel (I) durch ein Anion kompensiert wird, vorzugsweise ausgewählt aus der Gruppe der Liganden F⁻, Cl⁻, Br, I⁻, CN⁻, SCN⁻, OCN⁻, PF₆⁻, Zn₂Cl₆²⁻, BF₄⁻, NO₂⁻, NO₃⁻, HSO₄⁻, SO₄²⁻, H₂PO₄⁻, HPO₄²⁻, PO₄³⁻, OH⁻ und CH₃COO⁻.

2. Die Verbindung der allgemeinen Formel (I) nach Anspruch 1, wobei R¹, R², R³, R⁴, und R^{m} unabhängig voneinander aus der Gruppe bestehend aus H, -CH₃, und -CH(CH₃)₂ ausgewählt sind.

3. Die Verbindung der allgemeinen Formel (I) nach einem der vorhergehenden Ansprüche, wobei die Struktureinheit (IV) 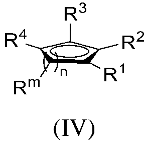 wird ausgewählt aus der Gruppe bestehend aus Cyclopentadien, Pentamethylcyclopentadien, Benzol, p-Cymol, 1,3,5-Trimethylbenzol und Hexamethylbenzol.

4. Die Verbindung der allgemeinen Formel (I) nach einem der vorhergehenden Ansprüche, wobei die Verbindung ausgewählt ist aus der Gruppe bestehend aus: 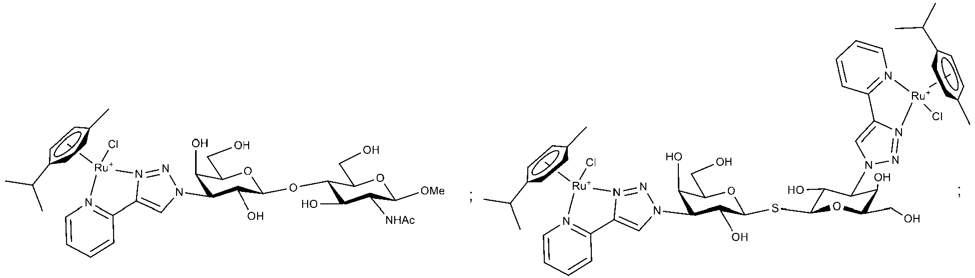 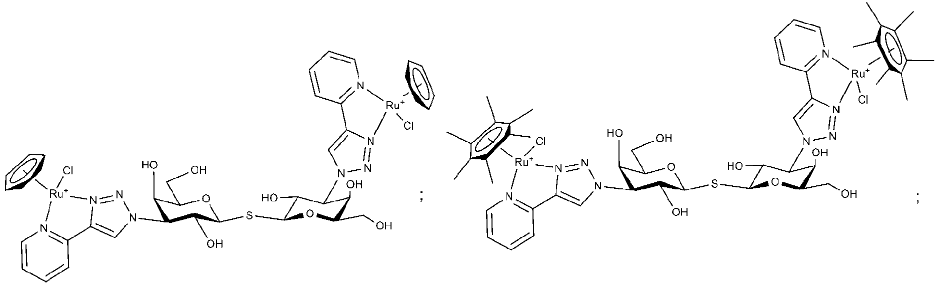 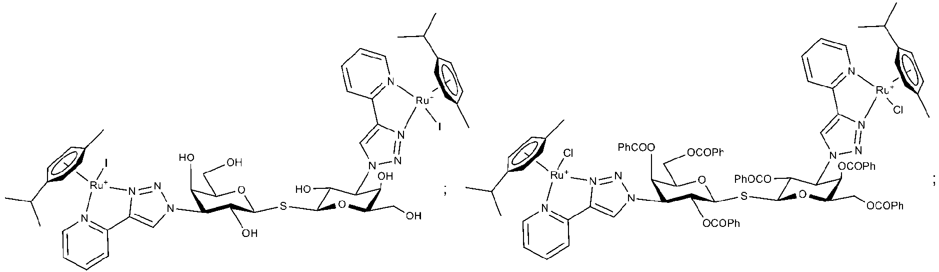 wobei die positive Ladung der Verbindung durch ein Anion kompensiert wird, das ausgewählt ist aus der Gruppe bestehend aus F⁻, Cl⁻, Br⁻, I⁻, CN⁻, SCN⁻, OCN⁻, PF₆⁻, Zn₂Cl₆²⁻, BF₄⁻, NO₂⁻, NO₃⁻, HSO₄⁻, SO₄²⁻, H₂PO₄⁻, HPO₄²⁻, PO₄³⁻, OH⁻ und CH₃COO⁻.

5. Verfahren zur Herstellung einer Verbindung der allgemeinen Formel (Ia): 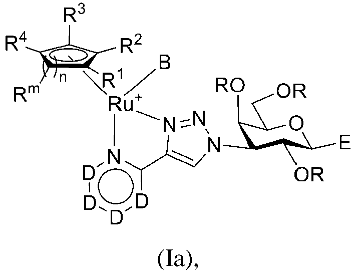
wobei B, D, R, R¹, R², R³, R⁴ und R^{m} wie in Anspruch 1 definiert sind;
und wobei E ist 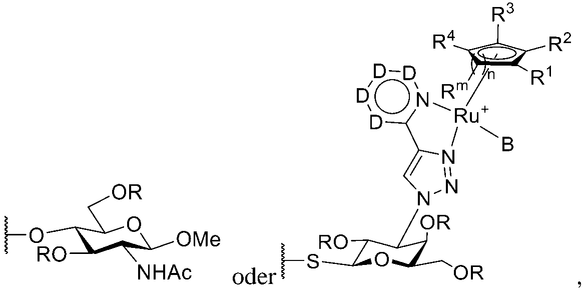
**gekennzeichnet dadurch, dass** es die folgenden Schritte umfasst:
(a) Huisgen-Cycloaddition des Azid-Vorläufers der allgemeinen Formel (V) 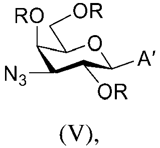
wobei R wie in Anspruch 1 definiert ist, vorzugsweise ist R H oder CH₃C(=O)-; und
A' ist 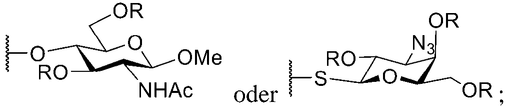
mit dem Ethinylderivat eines sechsgliedrigen stickstoffhaltigen Heterocyclus der allgemeinen Formel (VII), 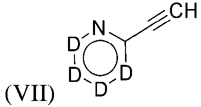
wobei D wie in Anspruch 1 definiert ist
was zur Bildung eines Zwischenprodukts der allgemeinen Formel (VIa) führt 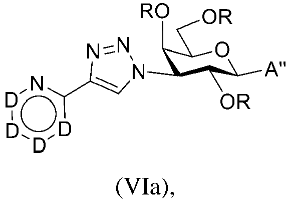
wobei R und D wie in Anspruch 1 definiert sind,
und A" ist 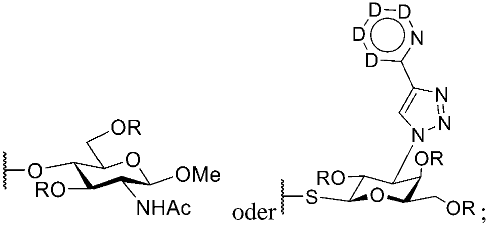
b) falls R nicht H ist, optionaler Schritt der Hydrolyse der OR-Gruppen zur Bildung eines Zwischenprodukts der allgemeinen Formel (VIb) 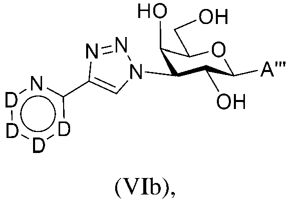
wobei D wie in Anspruch 1 definiert ist, und
A‴ ist 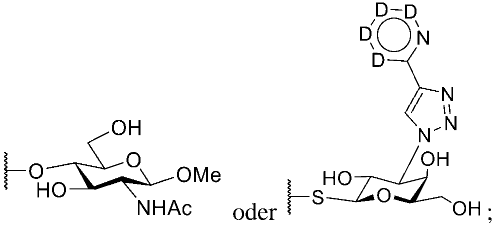
(c) eine Reaktion der Verbindung der allgemeinen Formel (VIb) mit einem Rutheniumkomplex der allgemeinen Formel [RuLB₂]₂, wobei L die in Anspruch 3 definierte Struktureinheit (IV) und B wie definiert in Anspruch 1 ist, zur Bildung der Verbindung der allgemeinen Formel (Ia).

6. Verfahren nach Anspruch 5, wobei zwischen Schritt (a) oder, falls vorhanden, Schritt (b) und Schritt (c) die Verbindung der allgemeinen Formel (VIb) mit einem Acylchlorid der Formel R'-C(=O)Cl zu einem Zwischenprodukt der allgemeinen Formel (VIc) reagiert 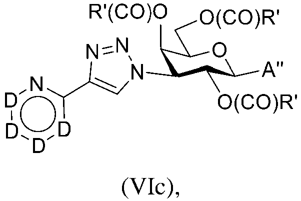
wobei R' und D wie in Anspruch 1 definiert sind, und
A" ist 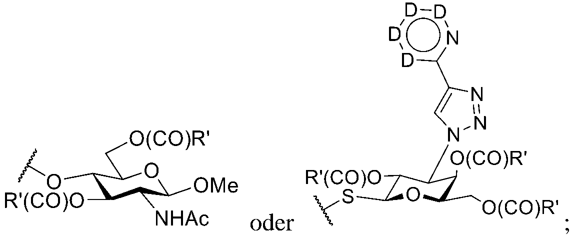
anschließend wird Schritt (c) mit der genannten Verbindung der allgemeinen Formel (VIc) durchgeführt, um die Verbindung der allgemeinen Formel (Ia) zu erhalten.

7. Verwendung der Verbindung der allgemeinen Formel (I) nach einem der vorhergehenden Ansprüche 1 bis 4 als analytische Sonde zur *In-vitro*-Bestimmung der Affinität von Liganden zu Galektinen, insbesondere zu Galektin-1.

8. Die Verbindung der allgemeinen Formel (I) nach einem der vorhergehenden Ansprüche 1 bis 4 zur Verwendung als Arzneimittel.

9. Die Verbindung der allgemeinen Formel (I) nach einem der vorhergehenden Ansprüche 1 bis 4 zur Verwendung in der Behandlung von Erkrankungen, die mit einer Galectin-Überexpression einhergehen, insbesondere einer Galectin-1-Überexpression, vorzugsweise ausgewählt aus der Gruppe Krebs, fibrotische Erkrankungen und HIV-1-Infektion.

10. Die Verbindung der allgemeinen Formel (I) nach einem der vorhergehenden Ansprüche 1 bis 4 zur Verwendung in der therapeutischen Hemmung von Galectinen *in vivo.*

11. Verwendung der Verbindung der allgemeinen Formel (I) nach einem der vorhergehenden Ansprüche 1 bis 4 zur Hemmung von Galectinen *in vitro,* insbesondere zur Hemmung von Galectin-1.

## Revendications

1. Un composé de formule générale (I) 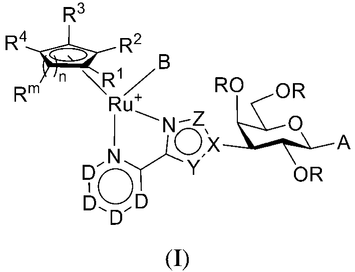 où :
R¹, R², R³, R⁴ et R^{m} sont sélectionnés indépendamment dans le groupe constitué de H et les groupes alkyles en (C₁ à C₆) ;
n est 1 ou 2 ;
X et Z sont N ;
D et Y sont CH ;
R est indépendamment H ou un groupe acyle de formule générale (G) 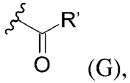
où R' est sélectionné dans le groupe comprenant les groupes alkyles en C₁-C₆, cycloalkyles en C₃-C₈ et aryles en C₆-C₁₂ ;
A est sélectionné dans le groupe constitué de les groupes de formules générales (II) et (III) 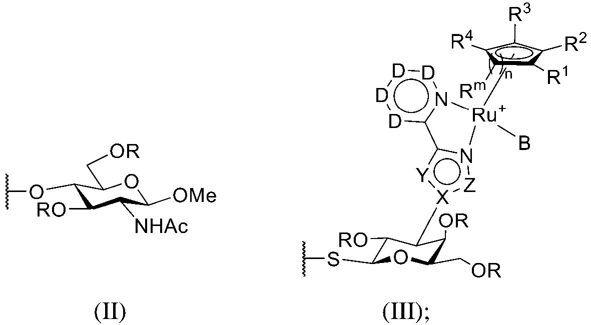
B est sélectionné dans le groupe comprenant les ligands monovalents chargés négativement et les ligands neutres, de préférence B est sélectionné dans le groupe comprenant F⁻, Cl⁻, Br⁻, I⁻, CN⁻, SNC⁻, OCN⁻, NO₂⁻, NO₃⁻, HSO₄⁻, OH⁻, CH₃COO⁻, CO, CO₂, CH₃CN, H₂O, NH₃, les N-ligands organiques monovalents et les ligands organophosphine ;
et dans lequel la charge positive du composé de formule générale (I) est compensée par un anion, de préférence sélectionné dans le groupe comprenant F⁻, Cl⁻, Br, I⁻, CN⁻, SCN⁻, OCN⁻, PF₆⁻, Zn₂Cl₆²⁻, BF₄⁻, NO₂⁻, NO₃⁻, HSO₄⁻, SO₄²⁻, H₂PO₄⁻, HPO₄²⁻, PO₄³⁻, OH⁻ et CH₃COO⁻.

2. Le composé de formule générale (I) selon la revendication 1, dans lequel R¹, R², R³, R⁴, et R^{m} sont sélectionnés indépendamment dans le groupe constitué de H, -CH₃, et -CH(CH₃)₂.

3. Le composé de formule générale (I) selon l'une quelconque des revendications précédentes, dans lequel l'unité structurale (IV) 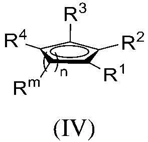 est sélectionnée dans le groupe comprenant le cyclopentadiène, le pentaméthylcyclopentadiène, le benzène, le p-cymène, le 1,3,5-triméthylbenzène et l'hexaméthylbenzène.

4. Le composé de formule générale (I) selon l'une quelconque des revendications précédentes, ledit composé étant sélectionné dans le groupe constitué de : 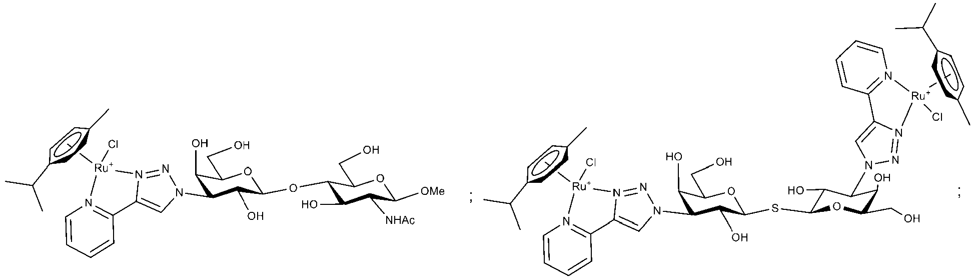 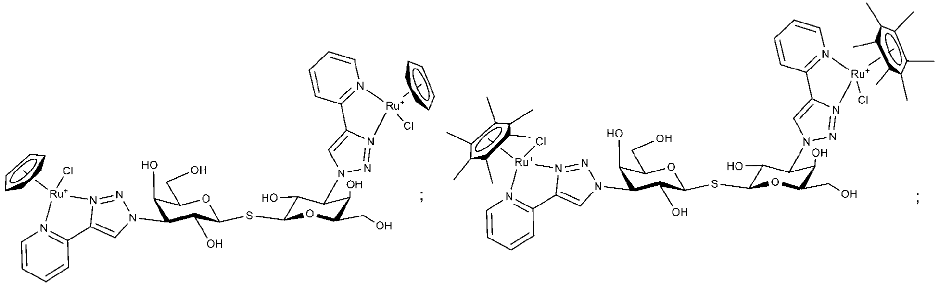 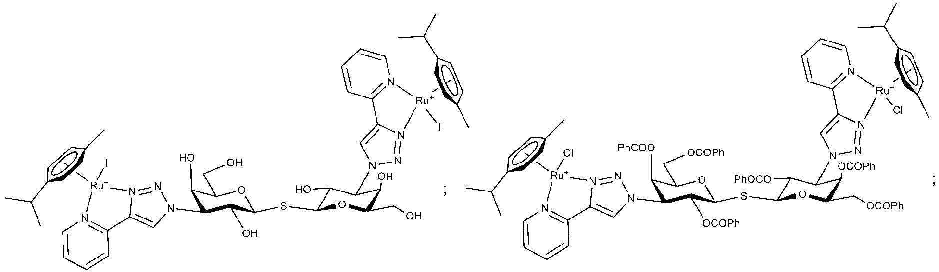 dans lequel la charge positive du composé est compensée par un anion sélectionné dans le groupe comprenant F⁻, Cl⁻, Br⁻, I⁻, CN⁻, SCN⁻, OCN⁻, PF₆⁻, Zn₂Cl₆²⁻, BF₄⁻, NO₂⁻, NO₃⁻, HSO₄⁻, SO₄²⁻, H₂PO₄⁻, HPO₄²⁻, PO₄³⁻, OH⁻ et CH₃COO⁻.

5. Un procédé de préparation d'un composé de formule générale (Ia) : 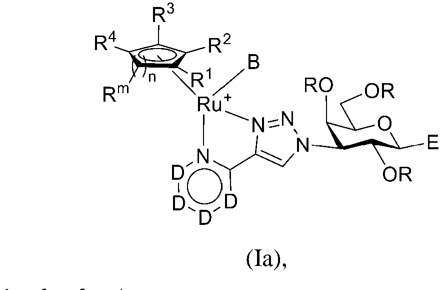
dans lequel B, D, R, R¹, R², R³, R⁴ et R^{m} sont tels que définis dans la revendication 1 ;
et dans lequel E est 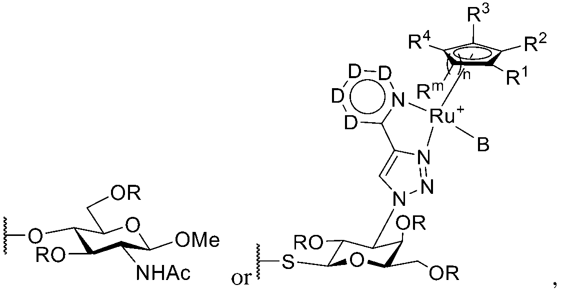
**caractérisé en ce qu'**il comprend les étapes suivantes :
(a) cycloaddition de Huisgen du précurseur azide de formule générale (V) 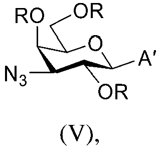
dans laquelle R est tel que défini dans la revendication 1, de préférence R est H ou CH₃C(=O)-; et
A' est 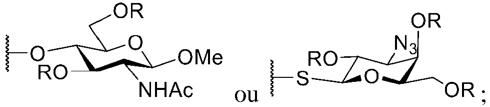
avec le dérivé éthynyle d'un hétérocycle azoté à six atomes de formule générale (VII), 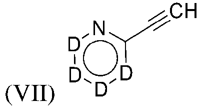
dans lequel D est défini dans la revendication 1,
ce qui entraîne la formation d'un intermédiaire de formule générale (VIa) 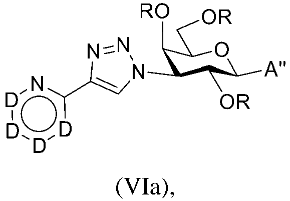
dans lequel R et D sont définis dans la revendication 1,
et A" est 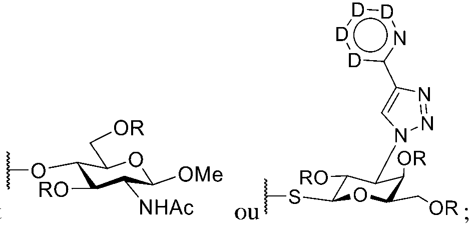
(b) si R n'est pas H, étape optionnelle d'hydrolyse des groupes OR puor former un intermédiaire de formule générale (VIb) 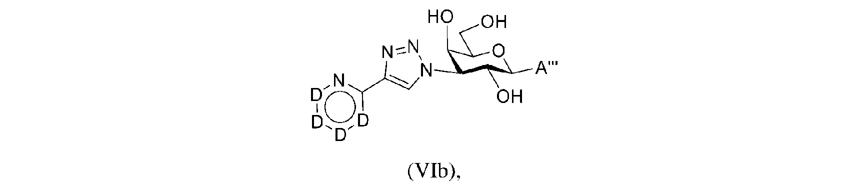
dans lequel D est tel que défini dans la revendication 1, et
A‴ est 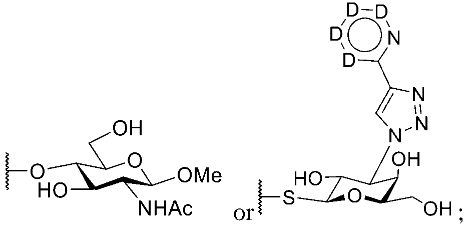
(c) une réaction du composé de formule générale (VIb) avec un complexe de ruthénium de formule générale [RuLB₂]₂, dans laquelle L est l'unité structurale (IV) telle que définie dans la revendication 3, et B est telle que définie dans la revendication 1, pour former le composé de formule générale (Ia).

6. Le procédé selon la revendication 5, dans lequel entre l'étape (a) ou l'étape (b), si présente, et l'étape (c), le composé de formule générale (VIb) réagit avec un chlorure d'acyle de formule R'-C(=O)CI pour former un intermédiaire de formule générale (VIc) 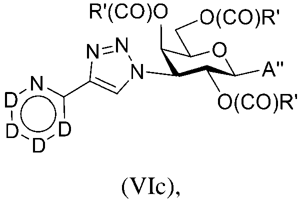
dans lequel R' et D sont définis dans la revendication 1, et
A" est 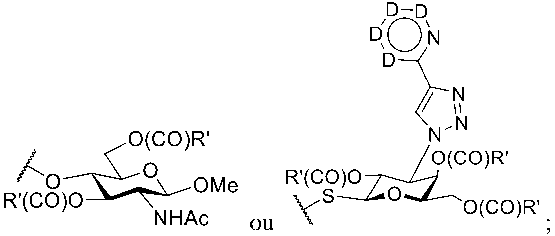
l'étape (c) est ensuite réalisée avec ledit composé de formule générale (VIc) pour donner le composé de formule générale (Ia).

7. Utilisation du composé de formule générale (I) selon l'une quelconque des revendications 1 à 4 précédentes, comme sonde analytique pour la détermination *in vitro* de l'affinité des ligands pour les galectines, en particulier pour la galectine-1.

8. Le composé de formule générale (I) selon l'une quelconque des revendications 1 à 4 précédentes, pour utilisation comme médicament.

9. Le composé de formule générale (I) selon l'une quelconque des revendications 1 à 4 précédentes, pour utilisation dans le traitement des maladies associées à une surexpression de galectines, en particulier associées à la surexpression de la galectine 1, de préférence sélectionnée dans le groupe comprenant le cancer, les maladies fibrotiques et l'infection par le VIH-1.

10. Le composé de formule générale (I) selon l'une quelconque des revendications 1 à 4 précédentes, pour utilisation dans l'inhibition thérapeutique des galectines *in vivo.*

11. Utilisation du composé de formule générale (I) selon l'une quelconque des revendications 1 à 4 précédentes pour l'inhibition des galectines *in vitro,* en particulier pour l'inhibition de la galectine-1.
